# EUROPÄISCHE PATENTSCHRIFT

(11) **EP 1 399 186 B1**
(45) Veröffentlichungstag und Bekanntmachung des Hinweises auf die Patenterteilung: **18.11.2009**
(21) Anmeldenummer: 02748771.9
(22) Anmeldetag: 12.06.2002
(51) Int. Cl.: A61K 39/395

(54) **ARZNEIMITTEL ZUR BEHANDLUNG VON TUMOREN UND DEREN METASTASEN UNTER VERWENDUNG EINES BINDEMOLEKÜLS GEGEN DAS BONE-SIALOPROTEIN**
MEDICAMENT FOR TREATING TUMOURS AND THEIR METASTASES USING A BINDING MOLECULE AGAINST BONE-SIALOPROTEIN
MEDICAMENTS A UTILISER DANS LE TRAITEMENT DE TUMEURS ET DE LEURS METASTASES UTILISANT UNE MOLECULE DE LIAISON CONTRE LA SIALOPROTEINE OSSEUSE

(30) Priorität: 13.06.2001 EP 01114388; 15.06.2001 DE 10128639
(43) Veröffentlichungstag der Anmeldung: 24.03.2004
(73) Patentinhaber: Armbruster Biotechnology GmbH, 64625 Bensheim (DE)
(72) Erfinder: ARMBRUSTER, Franz, Paul, 64625 Bensheim (DE); KARMATSCHEK, Markus, 64625 Bensheim (DE); NADER, Werner, Franz, 64625 Bensheim (DE); FORSMANN, Ulf, Jörg, 30625 Hannover (DE); PAULSSON, Mats, Inst.für Biochemie, Mediz.Fakultät, 50931 Köln (DE); BERGER, Martin, R., 64625 Bensheim (DE)
(74) Vertreter: Benedum, Ulrich Max
(86) Internationale Anmeldenummer: PCT/EP2002/006456
(87) Internationale Veröffentlichungsnummer: WO 2002/100899

(56) Entgegenhaltungen:
- WO-A-00/62065
- WUTTKE MARTINA ET AL: "Structural characterization of human recombinant and bone-derived bone sialoprotein: Functional implications for cell attachment and hydroxyapatite binding." JOURNAL OF BIOLOGICAL CHEMISTRY, Bd. 276, Nr. 39, 28. September 2001 (2001-09-28), Seiten 36839-36848, XP002230944 ISSN: 0021-9258
- FEDARKO N S ET AL: "FACTOR H BINDING TO BONE SIALOPROTEIN AND OSTEOPONTIN ENABLES TUMORCELL EVASION OF COMPLEMENT-MEDIATED ATTACK" JOURNAL OF BIOLOGICAL CHEMISTRY, AMERICAN SOCIETY OF BIOLOGICAL CHEMISTS, BALTIMORE, MD, US, Bd. 275, Nr. 22, 2. Juni 2000 (2000-06-02), Seiten 16666-16672, XP000938681 ISSN: 0021-9258
- FISHER L W ET AL: "ANTISERA AND CDNA PROBES TO HUMAN AND CERTAIN ANIMAL MODEL BONE MATRIX NONCOLLAGENOUS PROTEINS" ACTA ORTHOPAEDICA SCANDINAVICA, MUNKSGAARD, COPENHAGEN, DK, Bd. 66, Nr. SUPPL 266, 1995, Seiten 61-65, XP000937776 ISSN: 0001-6470
- WITHOLD W ET AL: "BONE SIALOPROTEIN IN SERUM OF PATIENTS WITH MALIGNANT BONE DISEASES" CLINICAL CHEMISTRY, AMERICAN ASSOCIATION FOR CLINICAL CHEMISTRY. WINSTON, US, Bd. 43, Nr. 1, 1997, Seiten 85-91, XP002923940 ISSN: 0009-9147
- DIEL I J ET AL: "Serum bone sialoprotein in patients with primary breast cancer is a prognostic marker for subsequent bone metastasis." CLINICAL CANCER RESEARCH: AN OFFICIAL JOURNAL OF THE AMERICAN ASSOCIATION FOR CANCER RESEARCH. UNITED STATES DEC 1999, Bd. 5, Nr. 12, Dezember 1999 (1999-12), Seiten 3914-3919, XP002230791 ISSN: 1078-0432

### SEQUENZPROTOKOLL

<110> Osteopep-Pharma GmbH
<120> Arzneimittel zur Behandlung von Tumoren und deren Metastasen
<130> HL-77234.003/UMB
<140>
   <141>
<150> EP 01114388.0
   <151> 2001-06-13
<160> 14
<170> PatentIn Ver. 2.1
<210> 1
   <211> 15
   <212> PRT
   <213> Homo sapiens
<400> 1
   Tyr Thr Gly Leu Ala Ala Ile Gln Leu Pro Lys Lys Ala Gly Asp
     1               5                  10                  15
<210> 2
   <211> 301
   <212> PRT
   <213> Homo sapiens
<400> 2
   Phe Ser Met Lys Asn Leu His Arg Arg Val Lys Ile Glu Asp Ser Glu
    1               5                  10                  15
   Glu Asn Gly Val Phe Lys Tyr Arg Pro Arg Tyr Tyr Leu Tyr Lys His
               20                  25                  30
   Ala Tyr Phe Tyr Pro His Leu Lys Arg Phe Pro Val Gln Gly Ser Ser
           35                  40                  45
   Asp Ser Ser Glu Glu Asn Gly Asp Asp Ser Ser Glu Glu Glu Glu Glu
       50                  55                  60
   Glu Glu Glu Thr Ser Asn Glu Gly Glu Asn Asn Glu Glu Ser Asn Glu
   65 70 75 80
   Asp Glu Asp Ser Glu Ala Glu Asn Thr Thr Leu Ser Ala Thr Thr Leu
   85 90 95
   Gly Tyr Gly Glu Asp Ala Thr Pro Gly Thr Gly Tyr Thr Gly Leu Ala
   100 105 110
   Ala Ile Gln Leu Pro Lys Lys Ala Gly Asp Ile Thr Asn Lys Ala Thr
   115 120 125
   Lys Glu Lys Glu Ser Asp Glu Glu Glu Glu Glu Glu Glu Glu Gly Asn
   130 135 140
   Glu Asn Glu Glu Ser Glu Ala Glu Val Asp Glu Asn Glu Gln Gly Ile
   145 150 155 160
   Asn Gly Thr Ser Thr Asn Ser Thr Glu Ala Glu Asn Gly Asn Gly Ser
   165 170 175
   Ser Gly Gly Asp Asn Gly Glu Glu Gly Glu Glu Glu Ser Val Thr Gly
   180 185 190
   Ala Asn Ala Glu Gly Thr Thr Glu Thr Gly Gly Gln Gly Lys Gly Thr
   195 200 205
   Ser Lys Thr Thr Thr Ser Pro Asn Gly Gly Phe Glu Pro Thr Thr Pro
   210 215 220
   Pro Gln Val Tyr Arg Thr Thr Ser Pro Pro Phe Gly Lys Thr Thr Thr
   225 230 235 240
   Val Glu Tyr Glu Gly Glu Tyr Glu Tyr Thr Gly Val Asn Glu Tyr Asp
   245 250 255
   Asn Gly Tyr Glu Ile Tyr Glu Ser Glu Asn Gly Glu Pro Arg Gly Asp
   260 265 270
   Asn Tyr Arg Ala Tyr Glu Asp Glu Tyr Ser Tyr Phe Lys Gly Gln Gly
   275 280 285
   Tyr Asp Gly Tyr Asp Gly Gln Asn Tyr Tyr His His Gln
   290 295 300
<210> 3
   <211> 12
   <212> PRT
<213> Homo sapiens
<400> 3
   Leu Gly Tyr Gly Glu Asp Ala Thr Pro Gly Thr Gly
    1               5                  10
<210> 4
   <211> 12
   <212> PRT
   <213> Homo sapiens
<400> 4
   Glu Thr Gly Gly Gln Gly Lys Gly Thr Ser Lys Thr
    1               5                  10
<210> 5
   <211> 12
   <212> PRT
   <213> Homo sapiens
<400> 5
   Phe Lys Gly Gln Gly Tyr Asp Gly Tyr Asp Gly Gln
    1               5                  10
<210> 6
   <211> 15
   <212> PRT
   <213> Homo sapiens
<400> 6
   Ile Gln Leu Pro Lys Lys Ala Gly Asp Ile Thr Asn Lys Ala Thr
    1               5                  10                  15
<210> 7
   <211> 15
   <212> PRT
   <213> Homo sapiens
<400> 7
   Tyr Thr Gly Leu Ala Ala Ile Gln Leu Pro Lys Lys Ala Gly Asp
   1 5 10 15
<210> 8
   <211> 17
   <212> PRT
   <213> Homo sapiens
<400> 8
   Gly Asp Ile Thr Asn Lys Ala Thr Lys Glu Lys Glu Lys Glu Ser Asp
   1 5 10 15
   Glu
<210> 9
   <211> 38
   <212> PRT
   <213> Homo sapiens
<400> 9
   Ser Glu Asn Gly Glu Pro Arg Gly Asp Asn Tyr Arg Ala Tyr Glu Asp
   1 5 10 15
   Glu Tyr Ser Tyr Phe Lys Gly Gln Gly Tyr Asp Gly Tyr Asp Gly Gln
   20 25 30
   Asn Tyr Tyr His His Gln
   35
<210> 10
   <211> 15
   <212> PRT
   <213> Homo sapiens
<400> 10
   Tyr Thr Gly Leu Ala Ala Ile Gln Leu Pro Lys Lys Ala Gly Asp
   1 5 10 15
<210> 11
   <211> 18
   <212> PRT
   <213> Homo sapiens
<400> 11
   Tyr Glu Ser Glu Asn Gly Glu Pro Arg Gly Asp Asn Tyr Arg Ala Tyr
   1 5 10 15
   Glu Asp
<210> 12
   <211> 9
   <212> PRT
   <213> Homo sapiens
<400> 12
   Leu Lys Arg Phe Pro Val Gln Gly Gly
   1 5
<210> 13
   <211> 34
   <212> DNA
   <213> Homo sapiens
<400> 13
   gcccgctagc cttctcaatg aaaaatttgc atcg 34
<210> 14
   <211> 38
   <212> DNA
   <213> Homo sapiens
<400> 14
   caatgactgc ggccgctcac tggtggtggt agtaattc 38

## Beschreibung

### GEBIET DER ERFINDUNG

Die Anmeldung betrifft Arzneimittel zur Behandlung und Bekämpfung von Tumoren und von Metastasen, die besonders häufig im Knochengewebe ansiedeln.

### HINTERGRUND DER ERFINDUNG

Es sind viele zur Zeit zahlreiche Arzneimittel in der Entwicklung, die Tumore und deren in Knochen streuende Metastasen bekämpfen sollen. Trotz aller medikamentösen Fortschritte gelten aber Knochenmetastasen nicht als heil- und therapierbar. Es gibt Versuche, durch Antikörper gegen Oberflächenantigene von Tumorzellen deren Metastasen zu bekämpfen. Knochenmetastasen sind aber trotzdem bei Tumoren der Mamma in 73% der Fälle und bei Tumoren der Prostata in 68% der Fälle die Todesursache. Für Tumoren von anderen Geweben gelten folgende Zahlen: Cervix 50%, Schilddrüse 42%, Blase 40%, Lunge 36%, Ovarien 9% und Kolon 6%.

Tumorzellen, die das sogenannte Bone-Sialoprotein exprimieren, haben die Besonderheit, dass sie bevorzugt in Knochengewebe einnisten und dort Metastasen bilden, besonders bei Tumoren der Prostata, Mamma, Lunge, Niere und Schilddrüse und weniger häufig bei malignen und semimalignen Tumoren. Das Bone-Sialoprotein (BSP) ist ein phosphoryliertes Knochen-Glykoprotein mit einer relativen Masse von ca. 80 kDa in der SDS-PAGE. Die cDNA für BSP kodiert für eine Peptidsequenz von ca. 33 kDa (Fisher L.W. et al. (1990), J. Biol. Chem., 265, 2347-51; US 5 340 934). BSP ist eines der wenigen Matrixproteine, deren Vorkommen auf mineralisierende Gewebe wie Knochen, Dentin und kalzifizierendem Knorpel beschränkt ist. Das BSP stellt ca. 10 bis 15 % der gesamten nicht-kollagenen Proteine in der Knochenmatrix. Es wird in der Regel von Zellen exprimiert, die an der Bildung von Dentin, Knochen und Knorpel beteiligt sind, bspw. von Osteoblasten, sich entwickelnden Osteozyten, hypertrophen Chondrozyten, Odontoblasten und Zementoblasten, aber auch von den Trophoblasten in der Placenta sowie von einigen Typen von Krebszellen, z.B. bei Lungen-, Brust-, Prostata-, Nieren-, Schilddrüsen- und Neuroblastoma-Primär- und Sekundärtumoren, beim Multiplen Myelom und in Knochenmetastasen. Der Grad der Expression von BSP durch den Tumor korreliert eng mit der Schwere der Krebserkrankung (Waltregny D. et al., Increased expression of bone sialoprotein in bone metastases compared with visceral metastases in human breast and prostate cancers, in J. Bone Miner. Res., 2000, 15(5), 834-43; Bellahcene, A. et al., Bone sialoprotein expression in primary human breast cancer is associated with bone metastases development, in J. Bone Miner. Res., 1996, 11, 665-670; Waltregny, D. et al., Prognostic value of bone sialoprotein expression in clinically localized human prostate cancer, in Journal of the National Cancer Institute, 1998, 90, 1000-1008; Bellahcène, A. et al., Expression of bone sialoprotein in primary breast cancer is associated with poor survival, in Int. J. Cancer, 1996, 69, 350-353).

Das BSP soll als Adhäsionsmolekül die Anheftung und Ausbreitung von Zellen auf der Gewebematrix bewirken, da es *in vitro* Kristallisationskeime für biologisches Apatit bildet und *in vivo* an den Mineralisationen beteiligt ist. Das Ausschalten des BSP-Gens in Knock-out-Mäusen führte zu keiner erkennbaren Störung der Skelettbildung und -funktion. In Tumoren wird BSP eine Beteiligung an der Mikrokalzifikation (Castronovo, V. et al., Evidence that breast cancer associated microcalcifications are mineralized malignant cells, in Int. J. Oncol., 1998, 12, 305-308) und der Besiedlung von Knochen durch metastasierende Tumorzellen zugeschrieben (Bellahcène, A. et al, Expression of bone sialoprotein in primary breast cancer is associated with poor survival, in Int. J. Cancer, 1996, 69, 350-353).

Die Höhe der Konzentration an BSP im Serum von Patienten mit primärem Karzinomen dient der Diagnostik, ob diese Patienten Knochenmetastasen besitzen oder solche wahrscheinlich vom primären Tumor ausgehen werden (Diplomarbeit von Frau Ina-Alexandra Meier, *Entwicklung eines Radioimmunoassays zur Bestimmung von Bonesialoprotein (BSP)*, 1996, Darmstadt, Fachhochschule, FB Chemische Technologie; Dissertation von Herrn Markus Karmatschek, *Isolierung von Bonesialoprotein aus humanem Knochen, Aufbau eines Radioimmunoassays zu dessen Messung im Serum*, 1996; FB Biologie der Technischen Hochschule Darmstadt; Diel I.J. et al., Elevated bone sialoprotein in primary breast cancer patients is a potent marker for bone metastases; in Proceedings of ASCO, 1998, 17, Abstract 461; Diel I.J. et al., Serum bone sia/oprotein in patients with primary breast cancer is a prognostic marker for subsequent bone metastasis, in Clin. Cancer Res., 1999, 5, 3914-19; DE 198 13 633; DE 198 21 533; WO 99/50666).

Freies BSP wird aber in Körperflüssigkeiten vom Komplementfaktor-H mit hoher Affinität gebunden. Zudem kann das BSP an verschiedene Rezeptoren binden. So wurden gegen verschiedene Peptidteilstrukturen des BSP (Fisher, L.W. et al., Antisera and cDNA probes to human and certain animal model bone matrix noncollagenous proteins. Acta Orthop Scand Suppl. 1995, 266, 61-655), gegen rekombinantes BSP (Stubbs JT 3rd et al., et al., Characterization of native and recombinant bone sia/oprotein: delineation of the mineralbinding and cell adhesion domains and structural analysis of the RGD domain. J. Bone Miner. Res. 1997, 12(8), 1210-22) und gegen aus Knochen isoliertes BSP Antikörper im Kaninchen hergestellt, die sämtlich BSP in Humanserum nicht erkennen. Das größere Faktor-H-Molekül mit 150 kDa maskiert vermutlich das kleinere BSP (von ca. 65 kDa) so, dass Antikörper nicht binden können. Zudem liegt Faktor-H im Serum im Überschuss vor (0,5 mg Faktor-H / mL im Vergleich zu BSP mit < 20 ng / ml Serum beim gesunden Menschen und max. 160 ng/ ml bei Tumorpatienten). Es wird behauptet, dass der immunologische Direktnachweis von BSP in Körperflüssigkeiten wegen der Bindung an den Faktor-H ohne reduzierende Probenaufbereitung unmöglich ist und möglicherweise Trophoblasten und BSP-produzierenden Tumorzellen hierdurch vor einem Angriff des Immunsystems geschützt werden, denn der Faktor-H gehört zum Komplementsystem und bewirkt bekanntlich die Hemmung des alternativen Weges zur Komplementlyse (Fedarko N.S. et al., Factor H binding of bone sialoprotein and osteopontin enables tumor cell evasion of complement-mediated attack, in J. Biol. Chem., 2000, 275, 16666-16672; WO 00/062065). Ferner kann das BSP über die eigene Erkennungssequenz (Arginin-Glycin-Aspartat, RGD) spezifisch an die Integrinrezeptoren auf Zelloberflächen binden. Bei einer Expression von BSP sollen dann die Tumorzellen den Faktor-H im Blut und in den Gewebsflüssigkeiten an ihre Zelloberfläche binden bzw. um sich herum konzentrieren. Ein derartiger BSP-Schutz vor dem Komplementsystem des Blutes der Mutter wird auch für die Trophoblasten in der Placenta vermutet (Fedarko N.S. et al., Factor H binding of bone sialoprotein and osteopontin enables tumor cell evasion of complement-mediated attack, in J. Biol. Chem., 2000, 275, 16666-16672; WO 00/62065). Eine Funktion des BSP wird weiterhin bei der Angiogenese vermutet. Neben der Adhäsion von Osteoklasten und Osteoblasten an die Knochenmatrix - durch das Binden der RGD-Erkennungssequenz in der Matrix an die alpha(v)beta(3) Integrin-Rezeptoren auf der Zellwand - wird auch beobachtet, dass die Adhäsion, Ausbreitung und Orientierung der Endothelzellen vermutlich von BSP vermittelt wird. Die Blutgefäßbildung um einen Tumor geht nämlich einher mit der BSP-Expression in den Tumorzellen (Bellahcène A et al, *Bone sialoprotein mediates human endothelial cell attachment and migration and promotes angiogenesis*, in Circ. Res. 2000, 86(8), 885-91)

Diese Eigenschaften machen das BSP somit zu einem Ausgangspunkt für Medikamente aller Art. So kann die Bindung von BSP über die RGD-Sequenz an Vitronectin- bzw. Integrin-Rezeptoren von Tumor- und Epithelzellen durch Antagonisten gehemmt werden (US 6 069 158; US 6 008 213; US 5 849 865; van der Pluijm et al., Bone-sialoprotein peptides are potent inhibitors of breast cancer cell adhesion to bone in vitro, in Cancer Res., 1996, 56, 1948-1955). EP 1 084 719 A1 lehrt eine pharmazeutische Zusammensetzung mit BSP als Wirksubstanz zur Unterstützung der Reparatur von geschädigten Knochen- und Bindegewebe. WO 94/13310 lehrt eine Zusammensetzung mit einem BSP-Bindeprotein aus Staphylococcus *aureaus* als Wirkstoff. WO 00/36919 offenbart regulatorische Elemente zur zielgerichteten Kontrolle bzw. Unterdrückung der Expression von BSP in Tumor- und Bindegewebszellen, welche die Kalzifizierung fördern. Allgemein sind also die Stoffe der Regulation des Zellwachstums und der Zellmigration diagnostisch und therapeutisch besonders interessant. Es gibt aber noch sehr viele *unbekannte* Faktoren, die das Krebswachstum steuern, wobei Primär- und Sekundärtumoren und besiedelte Organe interagieren. Wichtige Stufen sind hierbei die Invasion, die Adhäsion, die Migration und die Zellteilung der Tumorzellen. Neben Matrixmetalloproteinasen spielen hier Adhäsionsmoleküle und chemotaktische Faktoren eine besondere Rolle. Ein Medikament zur Bekämpfung und auch zur Heilung von Knochenmetastasen auf Grundlage von Antikörpern und Bindemolekülen gegen BSP ist nicht bekannt. Es ist auch nicht bekannt, dass das BSP aus Tumorzellen von dem BSP aus normalen gesunden Zellen verschieden ist.

### ZUSAMMENFASSUNG DER ERFINDUNG

Gegenstand der Erfindung ist ein in Anspruch 1 definiertes Arzneimittel zur Therapie von Tumoren und deren Metastasen, die sich bevorzugt im Knochengeweben ansiedeln, umfassend als Wirkstoff mindestens ein Bindemolekül, das an Bone-Sialoprotein oder einem Fragment hiervon in Serum oder Plasma bindet. Der Wirkstoff ist bevorzugt ein Antikörper oder ein Aptamer oder Spiegelmer (Noxxon, Berlin, DE) auf DNA oder RNA-Basis und bindet im weiteren Sinn ein Molekül, das einem chemisch oder natürlich in der Glykosylierung veränderten Bone-Sialoprotein entspricht. Das Bindemolekül ist ein Antikörper oder ein Aptamer, das spezifisch Bone-Slaloprotein aus Tumorzellen bindet, oder auch die Bindungsstruktur von natürlichen BSP-Rezeptor oder des Faktor-H-Moleküls. Das Bindemolekül bindet an bzw. es kann hergestellt werden gegen in der Glykosylierung verändertes Bone-Sialoprotein aus Knochenmaterial, dessen Spender nicht zur normalen Glykosylierung von Knochenproteinen fähig war.

In einer besonders bevorzugten Ausführungsform enthält das Arzneimittel als Wirkstoff einen Antikörper oder eine Mehrzahl von Antikörper gegen das humane Bone-Sialoprotein (hBSP), wobei die Antikörper Epitope binden, die auf humanem Bone-Sialoprotein aus Tumorzellen vorhanden sind, dessen posttranslationale Glykosylierung im Bereich der Aminosäuren 120 bis 135 (SWISSPROT: SIAL_HUMAN, Acc.No. P21815, inkl. Signalsequenz), beinhaltend die Aminosäuren TGLAA, gegenüber normalem Bone-Sialoprotein aus Knochen verändert oder unvollständig ist. Das erfindungsgemäße Arzneimittel kann als Wirkstoff auch einen Antikörper und/oder ein Aptamer enthalten, erzeugt gegen ein hBSP-Epitop, umfassend die Aminosäuresequenz TGLAA oder YTGLAA und optional Zuckergruppen sowie ein Trägermolekül. Der Wirkstoff ist bevorzugt ein IgY-Antikörper aus Huhn. Der IgY-Antikörper aus Huhn kann auch ein entsprechend humaner oder humanisierter Antikörper sein. Weiterhin bevorzugt sind Arzneimittel, wobei das Bindemolekül als bispezifischer Antikörper noch ein zusätzliches Paratop beinhaltet, das vorzugsweise spezifisch für Epitope von CD3 ist. Der Wirkstoff kann auch ein Immunotoxin sein, das ein Konjugat aus Bindemolekül und einem Rest mit zytotoxischer Aktivität ist. Das Immunotoxin kann bspw. ein Konjugat sein, das die Ricin-A-Kette oder ein nicht bindendes Fragment" des Diphtherietoxins beinhaltet. Das Bindemolekül kann ferner mit einem Radionuklid gekoppelt sein, so dass das Arzneimittel auch zur Immunszintigraphie bzw. zur Lokalisation und Verlaufsbeobachtung der Knochenmetastasen eingesetzt werden kann.

Das erfindungsgemäße Arzneimittel kann zusätzlich mindestens einen Antikörper, Liganden oder Inhibitor enthalten aus der Gruppe mit Adhäslonsmolekolen, membranassoziierten Proteasen, Rezeptoren, die Chemotaxis vermitteln, Chemokinrezeptoren, Apoptoseinduzierenden Substanzen. Die Inhibitoren können so gewählt werden, dass sie zumindest teilweise BSP blockieren und dessen Funktion modulieren. Das erfindungsgemäße Arzneimittel ist dem Anwendungsgebiet nach somit besonders geeignet zur Behandlung von Tumoren aus der Gruppe mit Prostata-, Mamma-, Lungen-, Nieren- und Schilddrüsentumoren, Tumorerkrankungen des Blutsystems, des Lymphsystems, des Herz-Kreislauf-Systems, des Nervensystems, des Respirationstraktes, des Verdauungstraktes, des endokrinen Systems, der Haut einschließlich Anhangsgebilde, des Bewegungsapparates und des Urogenitaltraktes.

Es werden nun weitere Merkmale und Vorteile der Erfindung mit Bezug auf die Beispiele und die anliegenden Abbildungen beschrieben.

### KURZE BESCHREIBUNG DER ABBILDUNGEN

Es zeigt:
- Fig. 1: einen Westernblot mit tumor- und knochenspezifischen Isoformen des BSP;
- Fig. 2: einen Westernblot des Zellkulturüberstands von untransfizierten EBNA-293-zellen (Negativkontrolle) und transfizierter EBNA-293-Zellen mit den Expressionskonstrukten GST-EK-BSP und his₆-myc-EK-BSP unter Verwendung eines monoklonalen Maus-Anti-BSP-Antikörpers;

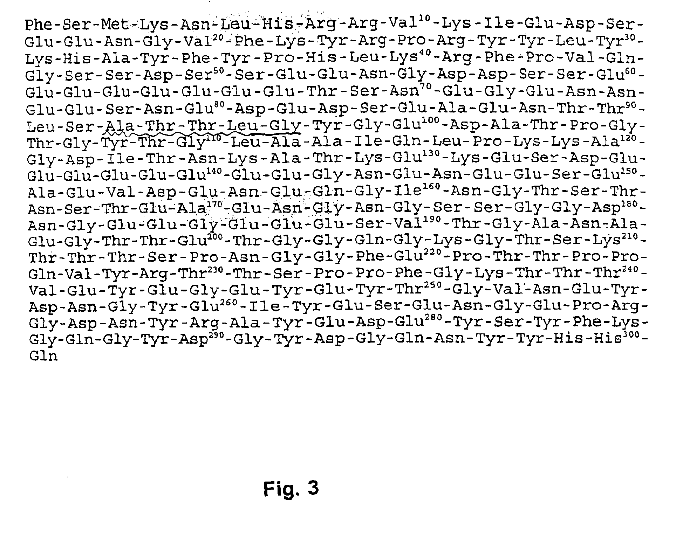

- Fig. 3: die Aminosäuresequenz von sekretiertem BSP (SEQ ID Nr. 2) nach Fisher et al (1991);

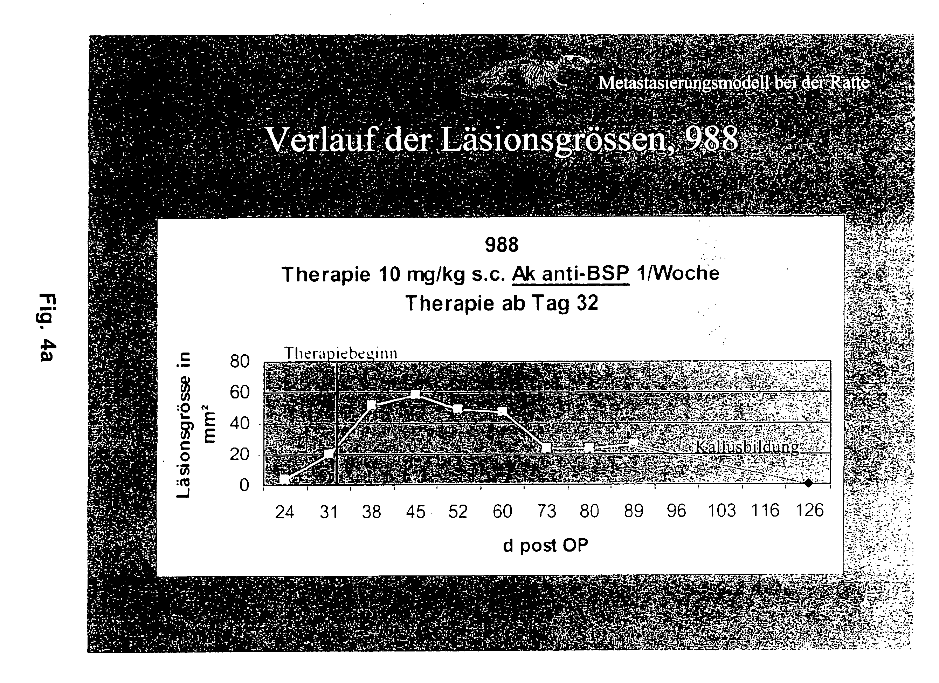

- Fig. 4a: Kurve mit Läsionsgrößen in Quadratmillimeter einer Knochenmetastase in der Tibia von der Ratte 988 über den Beobachtungs- und Therapiezeitraum;

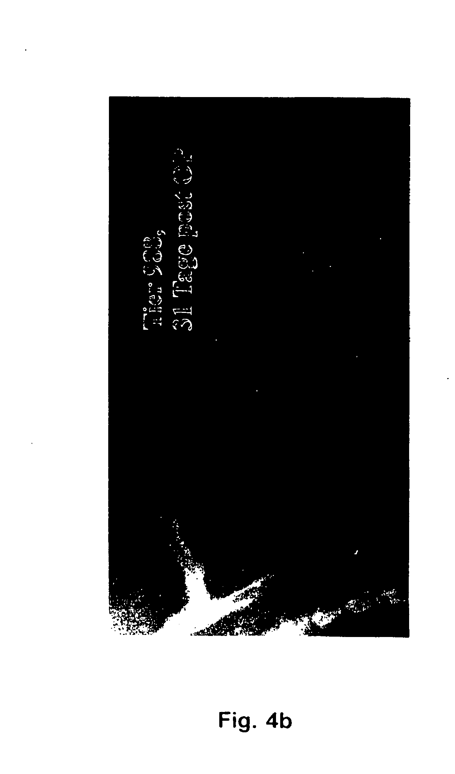

- Fig. 4b: Röntgenaufnahme 31 Tage post-OP von der Läsion in der Tibia vor Therapiebeginn;

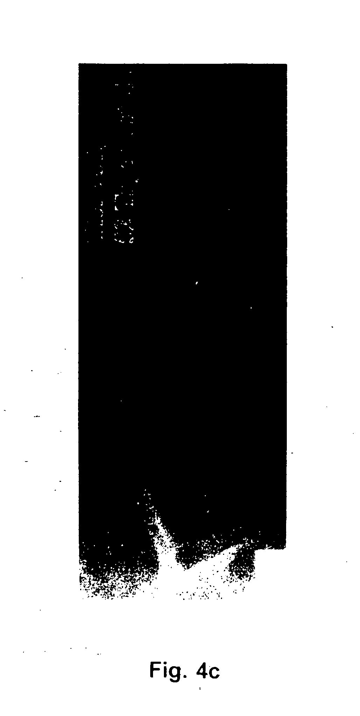

- Fig. 4c: Röntgenaufnahme 52 Tage post-OP von der Läsion mit noch fortschreitender Lyse des Knochen nach Therapiebeginn;

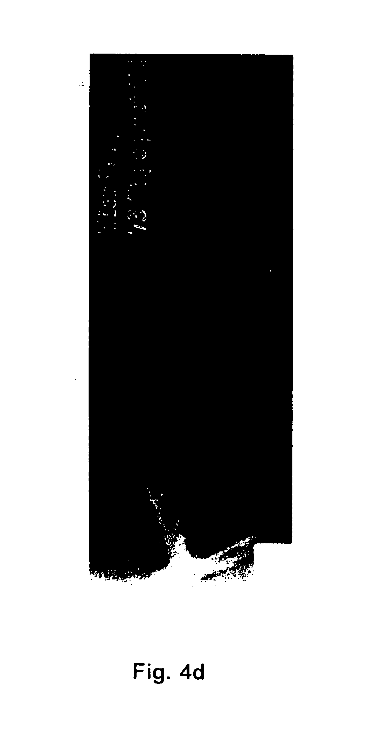

- Fig. 4d: Röntgenaufnahme 73 Tage post-OP von der sich rückbildenden Läsion;

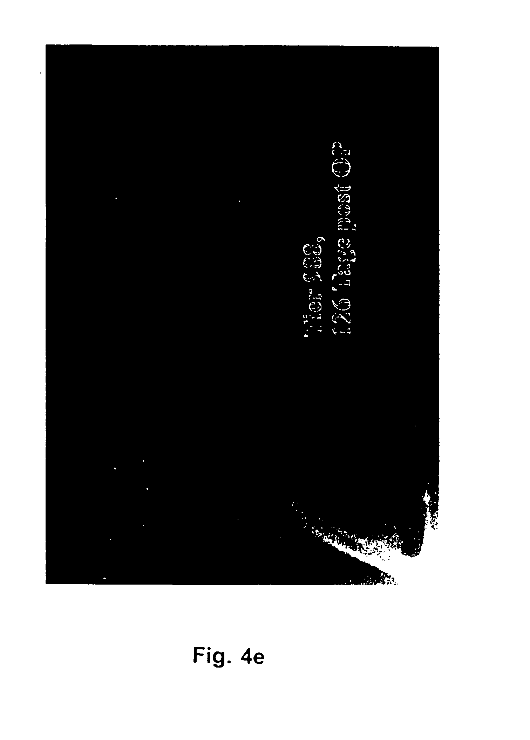

- Fig. 4e: Röntgenaufnahme 126 Tage post-OP von der verheilten Läsion:

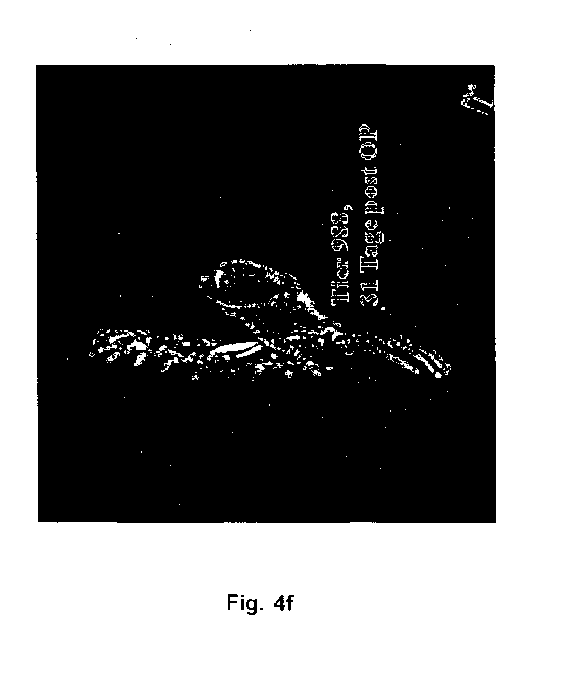

- Fig. 4f: CT-Rekonstruktion der Läsion 31 Tage post-OP;

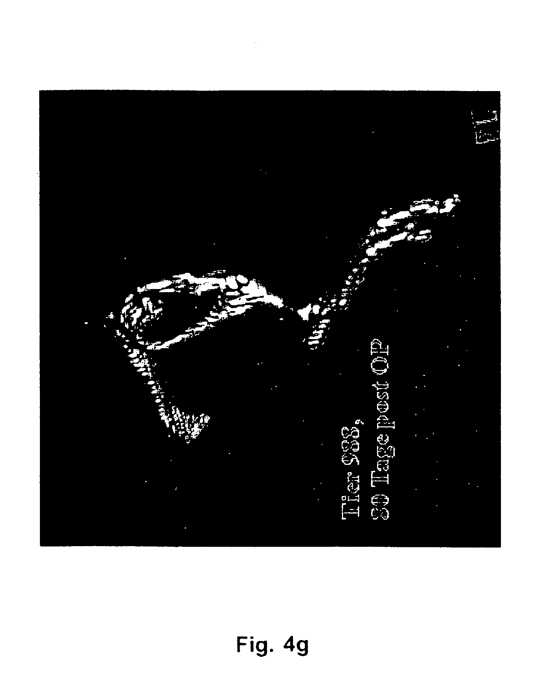

- Fig. 4g: CT-Rekonstruktion der sich rückbildenen Läsion 80 Tage post-OP;

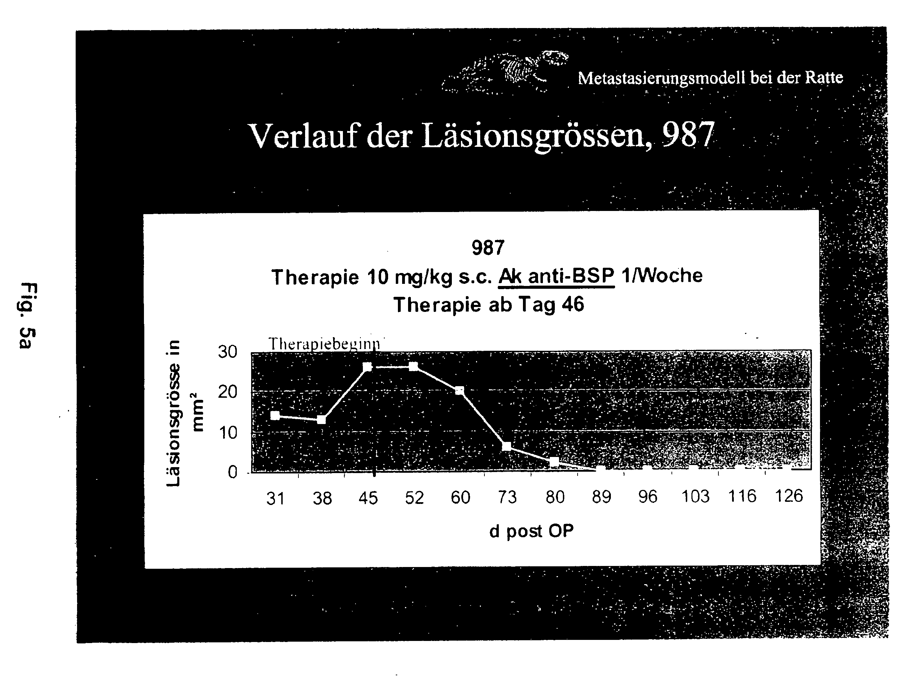

- Fig. 5a: Kurve mit Läsionsgrößen in Quadratmillimeter einer Knochenmetastase am distalen Femur von der Ratte 987 über den Beobachtungs- und Therapiezeitraum;

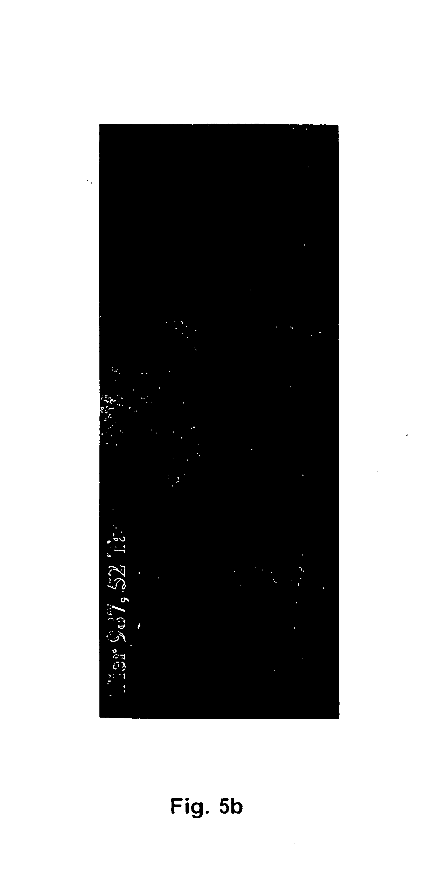

- Fig. 5b: Röntgenaufnahme 52 Tage post OP von der Läsion am distalen Femur;

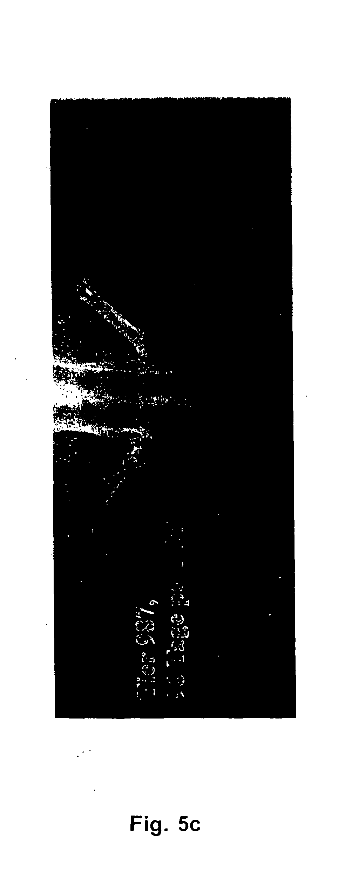

- Fig. 5c: Röntgenaufnahme 96 Tage post OP von der rückgebildeten Läsion und der Kallusbildung
.

### EINGEHENDE BESCHREIBUNG DER ERFINDUNG

Gegenstand der Erfindung ist somit ein Arzneimittel zur Therapie von Tumorerkrankungen, das den in Anspruch 1 definierten Wirkstoff enthält. In einer Ausführungsform ist das Bindemolekül ein Antikörper oder ein Aptamer auf der Basis von RNA oder DNA, das BSP in Gegenwart von Faktor-H erkennt. Besonders bevorzugte Bindemoleküle erkennen spezifisch BSP aus Tumorzellen. Das Arzneimittel kann durch folgende Substanzen verstärkt werden: Antikörper, Liganden oder Inhibitoren, die mit Adhäsionsmolekülen interagieren, mit membran-assoziierten Proteasen, oder mit Rezeptoren, welche eine Chemotaxis vermitteln wie beispielsweise die Chemokinrezeptoren, sowie Apoptose-induzierende Substanzen wie vorzugsweise Antikörper, Aptamere oder Proteine/Peptide, die aus natürlichen oder künstlichen Peptidbanken gewonnen sind. Eine spezifische Protein(Peptid)-Interaktion vorzugsweise mit unspezifischen Molekülen, die aus natürlichen Extrakten, aus synthetischen oder rekombinant hergestellten Bindungsproteinen sowie aus anderen Peptid-Protein-Banken gewonnen werden, reicht aber auch aus, um den Effekt der Apoptose von Tumorzellen herbeizuführen. Nach entsprechender Diagnostik kann eine spezifische Therapie angewendet werden. Hierbei wird überraschend bei Einsatz von Anti-BSP-Antikörper oder Bindeproteinen ein beschleunigter Eintritt des Tumorzelltodes (Apoptose) beobachtet.

Insbesondere so behandelbare Tumoren sind aus der Gruppe mit Mamma-, Prostata-, Lungen-, Nieren- und Schilddrüsentumoren sowie Tumorerkrankungen des Blutsystems, des Lymphsystems, des Herz-Kreislauf-Systems, des Nervensystems, des Respirationstraktes, des Verdauungstraktes, des endokrinen Systems, der Haut einschließlich Anhangsgebilde, des Bewegungsapparates und des Urogenitaltraktes einschließlich Niere.

Die Verabreichung der Bindeproteine und Antikörper erlaubt eine neue Therapie von Tumorerkrankungen auf Basis des BSP-Systems und Verstärkung unter Einschluss weiterer tumoroberflächen-assoziierten Proteomcluster. Das Arzneimittel beruht in der Verwendung von BSP-spezifischen Antikörpern, Aptameren, Liganden oder Inhibitoren gegen den Primär- oder Sekundartumor und streuende Metastasen, das heißt, zur Unterdrückung des Krebswachstums, inklusive Metastasierung. Das erfindungsgemäße Arzneimittel beruht auf der Feststellung, dass BSP durch autokrine, parakrine und endokrine Wege über die krankheitsspezifische Konstellation des Tumorzellproteoms auf spezifische Tumorzellen wirkt. Primär- und bestimmte Sekundärtumore werden in ihrem Adhäsions-, Migrations- und Proliferationsverhalten gesteuert. Durch diagnostischen Nachweis der lokal erhöht exprimierten und regulierten Faktoren sowie der Präsenz der BSP ergibt sich die Möglichkeit, das Krebswachstum, einschließlich Tumormetastasierung entscheidend zu unterdrücken oder vollständig zu verhindern.

Eine weitere Ausführungsform der Erfindung betrifft die Verwendung der erfindungsgemäßen Antikörper in pharmazeutischen Zusammensetzungen zur Behandlung von Tumoren und Metastasen. Die erfindungsgemäßen Antikörper sowie ihre Teilstrukturen oder Konjugate können durch Injektion oder über Zäpfchen appliziert werden und im Blut oder in Gewebeflüssigkeit frei zirkulierendes oder an Faktor-H gebundenes BSP binden und neutralisieren. Sollte eine bislang durch nichts belegte Schutzfunktion des Faktor-H-Komplexes gegen den alternativen Weg der Komplementaktivierung bestehen, so wird diese ausgeschaltet und die Tumorzellen für das Immunsystem angreifbar. Außerdem wird die angiogene Wirkung von BSP ausgeschaltet.

Für die Bindung an den Komplex aus Faktor-H und BSP müssen die Antikörper Epitope von BSP erkennen, welche nicht durch den Bindungspartner maskiert sind. Die Herstellung von solchen Antikörper war bisher nicht möglich. Die Erfindung stellt solche Antikörper zur Verfügung, weil die Antikörper gegen eine Isoform des gefalteten Bone-Sialoprotein (BSP) gerichtet sind und an Epitope binden, die nur von einem gefalteten Bone-Sialoprotein aus Tumorzellen gebildet werden, dessen Glykosylierungen im Bereich der Aminosäuren 120 bis 135 (mit Signalsequenz), umfassend die Aminosäuresquenz TGLAA oder YTGLAA, gegenüber dem normalem Bone-Sialoprotein aus Knochen verändert oder unvollständig sind oder fehlen. Normalerweise lassen sich keine spezifischen Antikörper gegen posttranslationale oder komplexe Zuckerstrukturen auf Proteinen erhalten, denn derartige Zuckerstrukturen werden in gleicher Weise und Form auf vielen verschiedenen Proteinen angefügt. Entsprechend reagieren Antikörper gegen bestimmte Zuckerstrukturen mit vielen verschiedene Proteinen und gelten dann in der Regel als unspezifisch und wertlos. Anders beim Bone-Sialoprotein aus Tumorzellen. Die veränderte bzw. fehlende Zuckerstruktur bewirkt eine andere Faltung des Bone-Sialoproteins und schafft neue Epitope, an denen sowohl Aminosäuren bzw. Peptidstruktur als auch die vielfältigen verbliebenen Zuckerreste beteiligt sind. Diese Epitope sind aber charakteristisch für BSP aus entarteten Tumorzellen.

Antikörper gegen diese Epitope können erzeugt werden mit einem chemisch oder natürlich in der Glykosylierung veränderten BSP als Antigen, und gegebenenfalls durch Aufreinigung bzw. Absorption an der Isoform des Knochen-BSP. Bevorzugt werden die Antikörper hergestellt unter Verwendung von BSP aus Tumorzellen als Antigen. Nachdem das BSP aus Tumorzellen schwer hinreichenden Mengen isoliert werden kann, ist die gentechnische Expression von in der Glykosylierung verändertem BSP in Tumorzellen die Methode der Wahl. Es wurde auch gefunden, dass einige Patienten in der Glykosylierung verändertes BSP im Knochenmaterial enthalten. Das heißt, diese zumeist sehr alten und an schwerer Osteoporose leidenden Patienten produzierten ein BSP, das zumindest in Teilen nicht normal glykosyliert war. Auch dieses BSP ist prinzipiell als Antigen zur Gewinnung der erfindungsgemäßen Antikörper geeignet. Die Isolierung der partiell glykosylierten Isoform, welcher der Tumor-Isoform des BSP gleicht, kann analog beschriebener Verfahren erfolgen (Karmatschek M et al., Improved purification of human bone sialoprotein and development of a homologous radioimmunoassay, in Clin Chem. 1997, 43(11), 2076-82).

Die Antikörper können hergestellt werden in Maus, Meerschweinchen, Kaninchen, Hund Ziege, Schwein, Mensch, Esel oder Pferd, aber auch in allen Säugetieren. Besonders bevorzugt ist die Immunisierung von Vögeln, insbesondere Huhn, da sich hier wegen der großen abstammungsgeschichtlichen Unterschiede besonders leicht Antikörper gegen die Tumor-Isoform des BSP erhalten lassen. Zudem führt die Gegenwart IgY-Antikörper nicht zu einer Aktivierung des Komplementsystems, was wegen der möglichen Bindung zwischen Faktor-H und BSP problematisch sein kann. Die erfindungsgemäßen Antikörper erkennen die Tumor-Isoform des BSP in der Bindung mit Faktor-H.

Gegenstand der Erfindung sind somit Isoformen des BSP, spezifische Antikörper gegen die von Tumoren gebildeten Isoformen und ihre Nutzung für eine Antikörpertherapie oder auch zur Immunszintigraphie. Als Nebenwirkungen, die durch Anti-BSP Antikörper hergerufen werden, kommen in Frage: direkte und indirekte Schädigung der Knochen und des Zahnbeins durch Aktivierung des Immunsystems gegen die Knochenmatrix und Knochenzellen und/oder direkte Zerstörung, bei Verwendung von Konjugaten der Antikörper mit Zellgiften oder Radioisotopen. Weiterhin ist eine Immunszintigraphie mit Anti-BSP-Antikörpern undenkbar, welche an die Knochenmatrix binden. Die Matrix würde radioaktiv markiert und die Lokalisierung von Tumoren wäre unmöglich. Die für Tumor-BSP spezifischen Antikörper eignen sich in besonderer Weise für eine Tumortherapie und -Lokalisierung, da sie nicht oder nur in geringem Ausmaß an die Knochenmatrix oder an BSP-produzierende Zellen des Skeletts und des Zahnbeins binden.

In einer besonders bevorzugten Anwendung der Erfindung werden Antikörper zur Tumortherapie eingesetzt, welche spezifisch für Tumor-BSP sind und zusätzlich BSP im Komplex mit Faktor H erkennen. Solche Antikörper werden durch die Erfindung bereitgestellt. Nach Applikation von solchen spezifischen Antikörpern in Tumorpatienten wird im Blut und in Gewebsflüssigkeit vorhandenes freies und an Faktor H gebundenes Tumor-BSP markiert und damit der Schutz gegen die Komplementaktivierung aufgehoben. Somit werden Tumorzellen spezifisch für die Zerstörung durch das Immunsystem markiert (z.B. durch klassische Aktivierung der Komplementkaskade) und Nebenwirkungen wie z.B. durch Aktivierung des Immunsystems gegen die Knochenmatrix oder das Zahnbein vermieden.

Für die durch die Erfindung möglich gewordene Tumortherapie und Immunszintigraphie können beispielsweise polyklonale Antikörper verwendet werden, welche hergestellt werden können durch Immunisierung von Hühnern mit rekombinantem BSP oder aus Knochen isoliertem in der Glykosylierung verändertem BSP. Die Antikörper werden dann in bekannter Weise aus dem Eidotter isoliert und über Affinitätschromatographie aufgereinigt.

In einer weiteren Anwendung der Erfindung werden humane polyklonale Anti-BSP Antikörper aus dem Ei von transgenen Hühnern mit humansiertem Immunsystem isoliert.

Ebenfalls geeignet sind monoklonale Antikörper aus der Maus oder dem Huhn, welche die oben beschriebenen Bedingungen erfüllen und durch ein Screening gewonnen werden können. In einer speziellen Anwendung des Patentes werden hierzu die in Beispiel beschriebene monoklonale Zelllinie verwendet. Weiterhin geeignet sind durch Fragmente von Antikörpern wie z.B. proteolytisch oder gentechnisch gewonnene Fab-Fragmente..

Für die Tumortherapie eignen sich weiterhin oben beschrieben Antikörper oder Antikörperfragmente in Konjugation mit Zellgiften und Radioisotopen zur direkten Zerstörung von Tumorzellen nach Bindung an BSP auf der Zelloberfläche.

Besonders geeignet sind humanisierte poly- und monoklonale Antikörper, die BSP im Komplex mit Faktor H erkennen und nicht an BSP in der Knochenmatrix binden. Bei der Verwendung von Antikörpern der Maus und des Huhns ist zwar ein besonderer therapeutischer Effekt durch Bildung humaner Anti-Maus-Antikörper (HAMA) oder Anti-Huhn-Antikörper (HACA) zu erwarten. HAMAs und HACAs können eine Immunantwort des Organismus auf das Tumorantigen induzieren und verstärken. Bei der Bestimmung von Tumormarkern entstehen jedoch Interferenzen mit den HAMAs und HACAs, welche die in vitro Messmethode stören. Auf diese Weise kommt es zu falsch hohen Meßwerten für Tumormarker. Dies tritt nach Immunszintigraphie oder Immuntherapie mit entsprechenden Antikörpern auf, so dass eine korrekte Tumormarkerbestimmung erst nach Absorption der HAMAs oder HACAs in vitro erfolgen kann.

Diese Effekte können durch Verwendung von humanisierten Antikörpern ausgeschaltet werden. Polyklonale humanisierte Anti-BSP Antikörper können beispielsweise durch Immunisierung von transgenen Hühnern mit BSP gewonnen werden, bei denen in den embryonalen Stammzellen der Genbereich für den hühnerspezifischen Fc-Teil des Immunglobulin (IgY) durch einen humanspezifischen ausgetauscht wurde (US 5 340 740; US 5 656 479). Die humanisierten Antikörper werden dann in den Eiern der Hühner abgelegt und können aus dem Eidotter isoliert werden (Mohammed S.M. et al., Deposition of genetically engineered human antibodies into the egg yolk of hens. Immunotechnology, 1998, 4: 115-125)

Zur Herstellung von humanisierten monoklonalen Antikörpern können Hybridomazellen der Maus oder des Huhns mit geeigneten Anti-BSP Antikörpern nach Standardmethoden gewonnen werden und aus dem in diesen Zellen enthaltenem Genmaterial durch Rekombination humanisierte Antikörper entwickelt werden (US 5 585 089; US 5 565 332; US 5 225 539; US 5 693 761; US 5 585 089; US 5 530 101).

Das BSP kann mit der Gesamtsequenz SEQ ID No 1 und der Teilsequenz ID No. 2 in seiner Gesamtheit oder mit seinen spezifischen Epitopen zur Generierung von Antikörpern eingesetzt werden.

Bevorzugte BSP-Fragmente zur Herstellung spezifischer Antikörper sind:
SEQ ID NR : 1
   X-Y**T**GLAAIQLPKKAGD-Z
wobei das markierte T nicht oder unvollständig oder in anderer Form glykosyliert ist und X und Z für Aminosäure- bzw. Peptidrest von bis zu 30 Aminosäuren stehen. In SEQ ID Nr. 2 können folgende Variationen vorliegen: an Position 179 Gly → Val; Position 252 Val → Ala; Position 254 Glu → Asp; Position 279 Asp → Gly.

Zur Herstellung der Antikörper werden die Peptide, die normalerweise nicht immunogen sind, an das Trägerprotein KLH (Keyhole Limpet Hemocyanin) gekoppelt. Diese Kopplung kann durch NBS (N-Maleimidbenzoyl-N-hydroxysuccinimidester) über ein im Peptid terminal addiertes Cystein, oder direkt mittels Carbodiimid erfolgen.

Die Antikörper werden mit herkömmlichen Verfahren durch Immunisierung vorzugsweise von Hühnern, Kaninchen, Mäuse, Meerschweinchen, etc. gewonnen. Es können auch molekularbiologische Verfahren wie die rekombinante Herstellung der Antikörper eingesetzt werden. Die Antikörper werden dann mit gereinigt und galenisch aufbereitet. Es können auch Zellpräparationen, Zellextrakte sowie insbesondere Membranisolate aus überexprimierenden, künstlich transfizierten BSP-exprimierenden Zellen zur Generierung spezifischer Antikörper eingesetzt werden.

Die erfindungsgemäßen Arzneimittel können in geeigneten galenischen Applikationsformen, insbesondere in lyophilisierter, mit Mannit oder ähnlichen Zuckern aufgenommener Form in sterilen Ampullen zur Auflösung in physiologischer Kochsalzlösung und/oder Infusionslösung zur wiederholten Einzelinjektion und/oder Dauerinfusion in Mengen von 300 mg bis 30 mg reiner Antikörper oder BSP-Liganden pro Therapieeinheit verabreicht werden. Vorzugsweise wird das erfindungsgemäße Arzneimittel in einer galenischen Applikationsform, bei der das Medikament in biokompatiblen Mikrosphären eingesetzt wird, und über Aerosol, intravenöse oder subkutane Applikation systemisch oder lokal verabreicht.

Mit verschiedenen Routine-Verfahren ist feststellbar, dass die Tumorzellen bei Gabe von Agonisten, die an die entsprechenden Proteommoleküle binden, anti-apoptotisch, adhäsiv, mitotisch und chemotaktisch reagieren. Die Hemmung ihrer Erhaltung, Adhäsion, Mitose bzw. Migration wird durch vorherige Inkubation mit Antagonisten oder Antikörper bewirkt.

Bei Verwendung hochgereinigter Antikörper gegen BSP in Zellkulturen BSP-exprimierender Tumorzelllinien konnte festgestellt werden, dass diese bei *in vitro* Modellen in der Lage sind die Apoptose von Tumorzellen zu bewirken. Züchtet man Zelllinien oder entnommene Tumorzellen unter Benutzung der üblichen Zellkulturverfahren, wird deren Überlebenszeit *in vitro* durch Beigabe von BSP-Antikörpern, wenn auf deren korrespondierende Zelloberfläche BSP nachgewiesen wurde, stark reduziert. Dabei ist eine Apoptose einer großen Zahl dieser kultivierten Zellen zu beobachten. Auch bei *in vivo* Modellen kann man überraschenderweise eine Tumorzellabnahme durch Apoptose feststellen.

Des weiteren kann bei Versuchen in der Zellkultur bei Tumorzellen die BSP exprimieren ein Einsatz von spezifischen BSP-Antikörpern die komplementvermittelte Zelllyse, als auch die zellulärvermittelte Tumorzelllyse eingeleitet werden.

Da Nacktmäuse bzw. Nacktratten ein defizientes Immunsystem besitzen, kann das Metastasierungsverhalten in einem Wirtskörper in einem Nacktmaus/-ratten-Modell untersucht werden, ohne dass die zwischen Spezies bekannten Immunreaktion stattfinden und eine Abstoßung der Fremdzellen erfolgt. Nacktmäuse werden in an sich bekannter Weise mit Tumorzellen oder Tumor-Zelllinien, deren BSP-Expression bestimmt worden war, geimpft und die Metastasierung durch diese Zellen bei Behandlung mit BSP-Antikörpern und bei Behandlung mit BSP-Liganden überprüft. Dabei ergibt sich überraschenderweise, dass bei den gefundenen BSP-positiven Tumoren eine Metastasenbildung deutlich gehemmt oder verhindert wird, weil die Antikörpergabe zu einer Modulation des Tumorwachstums führt. Überraschenderweise ergibt sich ebenfalls, dass die durch Immunhistochemie analysierten Präparate eine spezifische Verteilung von BSP und anderen tumoroberflächen-assoziierten Proteomclustern im Tumor und tumorumgebenden Gewebe zeigen. Damit wurden weitere gezielte Eingriffsmöglichkeiten erkannt.

Es ergibt sich die Erweiterung des therapeutischen Ansatzes, insbesondere gegen weitere Cluster des Tumorzelloberflächenproteoms gerichtete Antagonisten additiv anzuwenden Eine Verstärkung dieser Effekte kann besonders durch eine Kombination von BSP-Antikörpern mit Antikörper, Liganden oder Inhibitoren die mit (1) Adhäsionsmolekülen, (2) membran-assoziierten Proteasen, oder (3) Rezeptoren die Chemotaxis vermitteln, wie beispielsweise Chemokinrezeptoren, interagieren, sowie (4) Apoptose-induzierende Substanzen wie vorzugsweise Antikörper oder Proteine/Peptide, die aus natürlichen oder künstlichen Peptidbanken gewonnen werden können, erreicht werden.

Um diese Befunde zu bestätigen, können auch Tumorzelllinien mit BSP stabil transfiziert werden. Nach Injektion dieser Zellen (bei denen BSP überexprimiert ist) in Tiere, siedeln sich solche Tumorzellen bevorzugt in der Knochenmatrix an. Solche modifizierten Zellen bilden damit insbesondere Metastasen in Knochengewebe, anhand derer das therapeutische Prinzip ebenfalls nachweisbar ist.

Die Erfindung wird nachfolgend anhand von Beispielen näher erläutert:

### Beispiel 1 - Charakterisierung von tumor und knochenspezifischen BSP-Isoformen im Westernblot

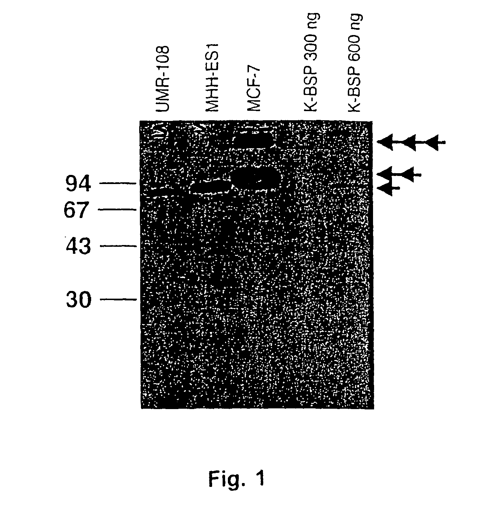

Serumfreie Überstände der humanen Osteosarkomzellinien UMR-108, MHH-ES1 und der Brustkrebszellinie MCF-7 (Östrogenrezeptor positiv) als auch aus Knochen gereinigtes humanes BSP (K-BSP) wurden mittels SDS-PAGE auf einem 10 % Gel unter reduzierenden und denaturierenden Bedingungen aufgetrennt und elektrophoretisch auf Nitrozellulose übertragen. Die Membran wurde mit dem monoklonalen Maus-Antikörper inkubiert. Die Detektion des BSP erfolgte über einen an Peroxidase gekoppelten Anti-Maus-Antikörper der Ziege und Chemoluminiszenz-Detektion auf einem Röntgenfilm. Das Ergebnis ist in Abb. 1 dargestellt. Molekulargewichte und Laufstrecken der Marker sind auf der linken Seite angegeben. Die einzelne und doppelte Pfeilspitze zeigen das unterschiedliche Laufverhalten von Knochen/Osteosarkom-BSP und MCF-7-BSP. Letzteres enthält zusätzlich eine hochmolekulare Bande (Tripelpfeil), die in den anderen Spuren abwesend ist. BSP aus einer Tumorzelllinie weist somit ein deutlich höheres Molekulargewicht als BSP aus Knochen und aus Osteosarkomzellinien auf, wobei außerdem eine zweite Isoform mit noch höherem Molekulargewicht zu beobachten ist.

### Beispiel 2 - Herstellung von polyklonalen Antiktirpern durch Immunisierung von Hühnern mit Knochen-BSP und BSP-Peptidteilstrukturen

Hühner und Kaninchen wurden mit BSP immunisiert, das nach dem von Karmatschek et al. (1997) beschriebenen Verfahren von Patienten isoliert worden war.

Aus den Eidottern und den Seren wurden polyklonale Immunglobuline isoliert und in einem ELISA-Verfahren gegen verschiedene Peptidteilstrukturen des BSP auf Bindung getestet. Tabelle 1 zeigt die Ergebnisse dieses Epitopmapping. Dabei wurden Peptidteilstrukturen aus der insgesamt 317 Aminosäuren langen Peptidsequenz des preproBSP (inkl. Leadersequenzen) chemisch synthetisiert, an eine Mikrotiterplatte gebunden und die Antikörper auf der Platte inkubiert. Der Test auf Bindung erfolgte nach Inkubation mit einem Konjugat von Peroxidase mit Anti-IgY- bzw Anti-Kaninchen-IgG-Immunglobulinen und nachfolgender Enzymreaktion durch Umsetzung eines Chromogens als Substrat.

**Tabelle 1**

| *Epitopmapping der gewonnenen anti-BSP-IgG und -IgY* | | | |
|---|---|---|---|
| Lage der Peptidteilstruktur im BSP | Aminosäuresequenz | Reaktionsstärke ELISA | |
| (Position inkl. Leader) | | IgY | Kaninche IgG |
| 112-123 | LeuGlyTyrGlyGluAspAlaThrProGlyThrGly | - | ? |
| 216-227 | GluThrGlyGlyGlnGlyLysGlyThrSerLysThr | - | ? |
| 300-311 | PheLysGlyGlnGlyTyrAspGlyTyrAspGlyGln | - | ? |
| 130-144 | IleGlnLeuProLysLysAlaGlyAspIleThrAsnLysAlaThr | +/- | + |
| 124-138 | TyrThrGlyLeuAlaAlaIleGlnLeuProLysLysAlaGlyAsp | - | ++ |
| 137-151 | GlyAspIleThrAsnLysAlaThrLysGluLysGluLysGlu-SerAspGlu | - | + |
| 280-317 | | ++ | + |
| Humanes Knochen-BSP | | +++ | +++ |

Die Ergebnisse zeigen, dass die gewonnenen Hühnerantikörper bevorzugt an die C-terminale Sequenz des BSP binden, während die Kaninchenantikörper über einen größeren Bereich binden.

Weiterhin wurden polyklonale Antikörper (A0001) durch Immunisierung von Kaninchen mit der Peptidteilstruktur TyrThrGlyLeuAlaAlaIleGlnLeuProLysLysAlaGlyAsp (Position 124-138) des BSP gewonnen, die bevorzugt an diese Peptidteilstruktur, aber auch spezifisch mit humanem Knochen-BSP reagieren.

Polyklonale Antikörper (AK_tBSP) jedoch, die durch Immunisierung von Kaninchen mit den Peptidteilstrukturen ThrGlyLeuAlaAla (Position 125-130) bspw TyrThrGlyLeuAlaAla (Position 124 bis 130) gewonnen wurden, das heißt nach Kopplung an Rinder-Thyreoglobulin als Träger, reagierten zwar mit der synthetischen Peptidteilstruktur, nicht aber mit human Knochen-BSP. Diese Antikörper erkennen überraschenderweise ausschließlich BSP aus Tumorzellen.

Für die Versuche wurden weiterhin die polyklonalen Antikörper A002 (erhalten von L.W.Fisher) und A003 (erhalten von Dr. van Ryden) verwendet. Diese Antikörper wurden nach Immunisierung mit den Peptiateilstrukturen TyrGluSerGluAsnGlyGluPro**ArgGlyAsp**AsnTyr-ArgAlaTyrGluAsp (A002) bzw. LeuLysArgPheProValGlnGlyGly gewonnen. Ersteres Peptid stammt vom C-Terminus des BSP (Position 278-295) des BSP und enthält die RGD **(ArgGlyAsp)**-EErkennungssequenz des BSP für Rezeptoren des Integrin-Typs. Letzteres Peptid stammt vom N-Terminus der BSP-Primärstruktur. Auch diese Peptide erkannten bevorugt die jeweiligen Teilstrukturen und reagierten spezifisch mit humanem Knochen-BSP.

### Beispiel 3 - Gewinnung von rekombinantem BSP aus Brustkrebszellen als Antigen

Aus dem Plasmid B6-5g (Fisher L.W. et al., Human bone sialoprotein. Deduced protein sequence and chromosomal localisation, in J. Biol. Chem., 1990, 265(4), 2347-51) wurde die komplette cDNA für humanes BSP (ohne Signalpeptid) mittels PCR amplifiziert und in den episomalen eukaryotischen Expressionsvektor pCEP-Pu (Kohfeldt E et al., Properties of the extracellular calcium binding module of the proteoglycan testican, in FEBS Lett. 1997, 414(3), 557-61) kloniert. Die Primer waren wie folgt:
Nhe I BSP (sense): 5'-GCCCGCTAGCCTTCTCAATGAAAAATTTGCATCG-3'
Not I BSP (antisense): 5'-CAATGACTGCGGCCGCTCACTGGTGGTGGTAGTAATTC-3"

Die mit den Primern eingefügte Nhe I- und Not I-Schnittstellen wurden für die Klonierung in den Expressionsvektor. PCEP-PU benötigt. Dieser Vektor ist zudem zur Erleichterung der Proteinaufreinigung am 5'-Ende der multiplen Klonierungsstelle mit verschiedenen tags (z.B. His, Myc, G8T) ausgestattet, Diese tags können nach Aufreinigung des Proteins mit einer Protease (z.B. Faktor X oder Enterokinase) abgespalten werden. Die Einhaltung des korrekten Leserahmens wurde mittels Sequenzierung überprüft.

Die Expressionskonstrukte wurden mittels Liposomen-vermittelter stabiler Transfektion (FUGENE™-Transfektionsreagenz der Firma Roche) unter anderem in folgende humane Zellinien eingeführt:
- die embryonale Nierenzellinie EBNA-293
- die Osteosarkomzellinien SAOS-2 und MG-63
- die humane Brustkrebszellinie MCF-7.

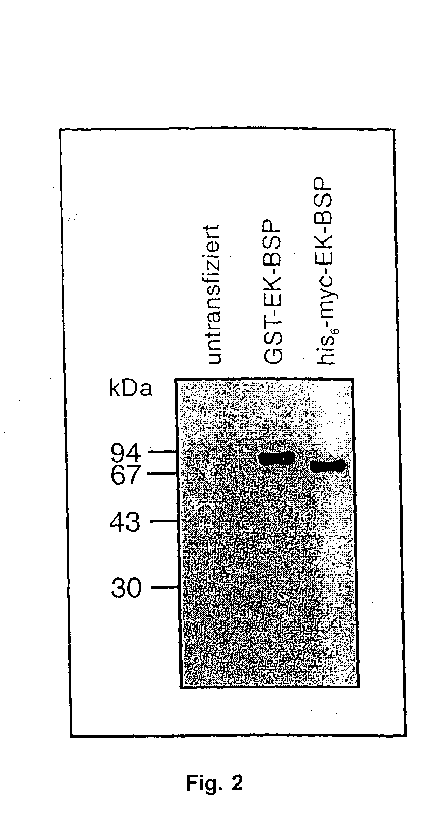

Eine rekombinante Expression wurde nur in MCF-7 und EBNA-293-Zellen erhalten (siehe Figur 2). Die Osteosarkomzelllinen exprimiert auch nach wiederholten Transfektionsversuchen nicht.

### Beispiel 4 - Analyse der Glykosylierung von rekombinantem BSP aus entarteten Zellen und Knochen-BSP

Transiente Zellen wurde 48 Stunden nach Transfektion zwei Tage in serumfreiem Medium kultiviert. Damit die Proteine im FCS die Aufreinigung des rekombinanten BSP nicht erschwerten, wurden BSP-exprimierende Zellen nach Erreichen der Konfluenz unter serumfreien Bedingungen kultiviert. Unter diesen Bedingungen konnten nur EBNA-293 Zellen länger als 2 bis 4 überleben. Die Expression des rekombinanten BSP wurde durch SDS-PAGE und Immunoblots kontrolliert.

Die Untersuchung von serumfreien Zellkulturüberständen ergab mit all diesen Zelllinien im Westernblot positive Signale sowohl in Bezug auf BSP wie auch auf die Anwesenheit der verschiedenen tags.

2,5 Liter serumfreier Kulturüberstand der transfizierten MCF-7 Zelllinie wurde über eine Sepharose™-Säule aufgereinigt und daraus 250 µg homogenes His-myc-EK-BSP gewonnen. Das so aufgereinigte Expressionsprodukt war partiell glykosyliert, besaß aber keine Glykosylierung an Threonin 125, das heißt dem Threonin in der BSP-Sequenz YT¹²⁵LPAA.

Für die Glykoanalytik wurden die N-Glykane vom rekombinanten BSP (rBSP) bzw. dem Knochen-BSP enzymatisch mit dem Peptid N-Glykosidase F (PNGase F, Roche) abgetrennt. Das Enzym bewirkt eine katalytische Spaltung allen N-Glykan-Typen von den Asparaginen. Für den Verdau wurden 20 bis 200 µg BSP mit Ethanol gefällt und das Fällungspellet in 1% SDS, β-Mercaptoethanol, 0,1 M EDTA 30 Minuten bei Raumtemperatur mit einem überschuß an Enzym inkubiert. Es folgte ein Verdau mit N-Glykosidase F über Nacht bei 37°C. Zum Entsalzen der N-Glykan-Lösung wurde der Verdau über eine 150 mg Carbonsäule (Carbograph SPE, Alltech) gegeben und die N-Glykane mit 25% aCN in 0,05% TFA eluiert.

Die O-Glykane wurde mittels wasserfreier Hydrazinolyse unter eines Kits (Oglycan relase kit, Glyco) vom BSP abgspalten. Hierzu wurden etwa 200 µg salzfreies BSP 24 Studen lyophilisiert, unter Argon-Schutzgas mit 50µl Hydrazin-Reagenz versetzt, gelöst und 5 Stunden bei 60°C inkubiert. Das Hydrazin wurde unter Vakuum abgezogen. Es folgt eine Re-N-Acetylierung der N-AcetylGruppen mit Essigsäureanhydrid.

Die N- und O-Glykane wurde mit dem Fluoreszenzfarbstoff 2-Aminobenzamid (Fluka) markiert und die 2-AB markierten Oligosaccharide sequenziell mit spezfischen terminalen Glykosidasen verdaut und mittels MALDI-TOF-Massenspektrometrie analysiert.

### Diskussion der Analytik

Die Aminosäuresequenz von humanem BSP enthält vier potentielle N-Glykosylierungsstellen an den Positionen 88 (NTT), 161 (NGT), 166 (NST) und 174 (NGS). Für O-Glykosylierungen ist keine vergleichbare Konsensussequenz bekannt. Alle identifizierten N-Glykan-Sturkutren waren sowohl auf dem aus Knochen isolierten BSP als auch auf dem rekombinanten EBNA-293 BSP zu finden. Unterschiede gab es jedoch im prozentualen Anteil der jeweiligen Strukturen an den gesamten N-Glykanen. So bestand der Hauptanteil der BSP-N-Glykane im Knochen aus triantennären Strukturen (58%) und in der EBNA-zelllinie aus tretraantennären Strukturen (48%).

Zur Lokalsierung der O-Glykosylierungsstellen von rekombinantem BSP wurden die O-Glykane durch sequenziellen Verdau des Proteins mit Neuraminidase, β-Galactosidase, und β-N-Acetylhexosaminidase bis auf das core-GalNAc entfernt. Das partiell deglykosylierte Protein wurde dann durch Behandlung mit Trpysin und V8-Protease in Peptidfragment gespalten. Mittels MALDA-TOF-Massenspektrometrie wurden die Massen der Peptide bestimmt und ein Teil der Peptide mittels PSD-MALDI-TOF-Massenspektrometrie sequenziert. Mit diesem Verfahren konnten acht O-Glykosylierungsstellen des rekombinanten BSP bestimmt werden, 5 auf dem Peptid 211-229 (TTTSP ... QVYR) und maximal drei auf dem Peptid zwischen AS 120 und AS 135 mit der Sequenz TGLAA.. Hiervon sind im rekombinanten BSP die Threonine in der Sequenz DATPGTG O-glykosyliert. Bei Knochen-BSP erfolgt eine dritte O-Glykosylierung. Bei rekombinantem BSP ist keine dritte Glykosylierungsstelle vorhanden. Vermutlich liegt diese Glykosylierungsstelle auf der TGLAA-BSP-Teilstruktur.

### Beispiel 5 - Herstellung von Anti-BSP-IgY aus Eidottern

Für die Aufreinigung größerer Mengen von Anti-BSP-IgY für die Therapie und Immunszintigraphie sind verschiedene Verfahren beschrieben. Bevorzugt wird das Verfahren von Akita und Nakai benutzt (Akita E..M et al., Comparison of four purification methods for the production of immunoglobulins from eggs laid by hens immunized with an enterotoxigenic E. coli strain, in J Immunol Methods. 1993, 160(2), 207-14).

Für die Eiproduktion wird eine Hochleistungsrasse wie "Lohmann Weiß" oder "Lohmann Braun" mit einer Leistung von 4,5 Eiern pro Woche und einer Produktion von über 10 mg spezifischen IgY pro Dotter verwendet. Die Immunisierung erfolgt mit aus menschlichem Knochen isoliertem oder rekombinantem BSP-Antigen in Freund'schen Adjuvans, wobei nach einer Grundimmunisierung mit ca. 0,1 mg BSP alle 6 Wochen Boosterinjektionen gesetzt werden. Ca. 30 % dieser Hühner reagieren normalerweise nicht auf die Immunisierung. Die Eier werden äußerlich mit Peressigsäure desinfiziert, dann aufgeschlagen und die Dotter vom Eiweiß abgetrennt. Die Dotter werden dann mit 5 bis 10 Volumina eiskaltem destilliertem Wasser zwischen pH 5 und 5,2 verquirlt und bei 2 bis 5 ° C über 2 bis 6 h inkubiert. Dabei sedimentieren die Dottergranula, die im wesentlichen aus Lipoproteinen bestehen. Der wässrige Überstand wird dann durch Filterpapier (z.B. Whatman No. 1) klar filtriert.

Aus diesem Überstand können die Anti-BSP-IgY direkt über Affinitätschromatographie homogen aufgereinigt werden. An eine mit Cyanogenbromid aktivierte Sepharose-4B-Säule wird aus menschlichem Knochen oder aus Kulturüberständen von rekombinanten humanen Zelllinien isoliertes BSP chemisch kovalent gebunden. Zur Bindung von 1 g IgY ist 0,5 g immobilisiertes BSP erforderlich (kovalent an ca. 5 ml Sepharose™ gebunden).

Das gebundene IgY wird über einen Säuregradienten eluiert und danach die Lösung neutralisiert. Diese Lösung muss dann entsalzt und die Antikörper konzentriert werden, was im Crossflow-Verfahren im großen Maßstab möglich ist (z.B. Amicon™ Spiralfilter SY100 mit einem Ausschluss von 100.000 Dalton).

### Beispiel 6 - Isolierung von Anti-BSP-IgY, das an den BSP-Faktor-H-Komplex bindet

Die geringe Reaktion der polyklonalen Hühnerantikörper mit BSP in der Knochenmatrix kann durch Selektion jener Antikörper ausgeschaltet werden, die mit BSP im Komplex mit Faktor H reagieren. Dazu wird entweder Faktor H oder aus Knochen isoliertes oder gentechnisch hergestelltes BSP an Cyanogenbromid aktivierte Sepharose 4 B chemisch kovalent gebunden und danach soviel BSP bzw. Faktor H auf die Säule aufgetragen und gebunden, dass sämtliche Liganden in der Matrix mit dem Partner komplexiert sind. Filtrierter Dotterextrakt wird dann auf dieser Affinitätssäule aufgetragen und wie in Beispiel 4 wird nun jene Antikörperfraktion gewonnen, die spezifisch an das freie Epitop im BSP-Faktor-H Komplex bindet.

### Beispiel 7 - Herstellung von humanen Anti-BSP Antikörpern in transgenen Hühnern

Anti-BSP-IgY weisen in der Humantherapie bzw. Diagnostik einige Schwächen auf. So sind Nebenwirkungen wie Fremdeiweißreaktionen zu erwarten und die biologische Halbwertszeit beträgt im Vergleich zu humanen Antikörpern nur 12 bis 24 Stunden. IgY aktiviert auch nicht das Komplementsystem.

Humane Antikörper gegen BSP können in speziellen transgenen Hühnern hergestellt werden, in denen durch Gene-Targeting die konstante Region für aviäres Immunglobulin in den für die Antikörperbildung verantwortlichen Genen durch die konstante Region für humanes Immunglobulin ausgetauscht wurde. Geeignete Huhnstammzellen und Vektorsysteme sind in den US-Patenten 5,340,740, Nr. 5,656,479 und Nr. 5,464,764 beschrieben. Nach Immunisierung mit BSP reagieren solche Hühner mit der Produktion von humanen Antikörpern im Ei.

### Beispiel 8 - Immunblotanalyse der Expression von BSP in menschlichen Brustkrebszellinien

Die Tumorzellinien MDA-MB-231 (Brustkrebszellinie, Östrogenrezeptor-negativ), MCF-7 (Brustkrebszellinie, Östrogenrezeptor-positiv) und T-47-D (Brustkrebszellinie, Östrogenrezeptor-positiv) wurden mit Immunpräzipitationspuffer extrahiert und BSP mit dem polyklonalen Antikörpergemisch A0001 aus Kaninchen gegen humanes BSP gefällt. Die Präzipitate wurden nach Denaturierung auf SDS-Gelen aufgetragen, die Elektrophorese durchgeführt und die Proteine auf Nitrozellulosemembranen übertragen. Danach erfolgte eine Immunfärbung mit dem anti-BSP-Kaninchenantiserum A001 und einem monoklonalen Maus-Anti-BSP-Antikörper (BSP 1.2), wobei als Zweitantikörper Peroxidasekonjugate von Antikörper der Ziege gegen Kaninchen IgG und gegen Maus IgG verwendet wurden. Auf beiden Blots A und B war die Bande des immunpräzipitieren BSP bei 70.000 Dalton deutlich zu erkennen.

Um die Anwesenheit bzw. Abwesenheit von BSP auf der Zelloberfläche von Tumorzellen zu zeigen, wurden die Zelloberflächen der Brustkrebszellinien MDA-MB-231 und MCF-7 biotinyliert, mit Immunpräzipitationspuffer extrahiert und BSP mit dem polyklonalen Antikörpergemisch A0001 aus Kaninchen gegen humanes BSP gefällt. Die Präzipitate wurden nach Denaturierung auf SDS-Gelen aufgetragen, die Elektrophorese durchgeführt und die Proteine auf eine Nitrozellulosemembran übertragen. Biotinylierte Proteine auf dieser Membran wurden dann mit einem Konjugat aus Peroxidase und Streptavidin mit dem ECL-System (Amersham) nachgewiesen.

Menschliche Brustkrebszellen der Linien T-47-D und MDA-MB-231 wurden mit und ohne vorherige Permeabilisierung mit einem anti-Schweine-BSP-Antikörper aus Kaninchen und einem mit Fluorescein konjugierten anti-Kaninchenantikörper der Ziege immunfluoreszent markiert. Fluoreszent markiertes BSP ist in beiden Zellinien nach Permeabilisierung zu erkennen Nur auf den T-47-D Zellen ließ sich auch ohne Permeabilisierung BSP über Immunfluoreszenz nachweisen.

### Beispiel 9 - Nachweis der BSP-Expression in Tumorzellen über RT-PCR

Aus den Tumorzellinien MDA-MB-231 (Brustkrebszellinie, Östrogenrezeptor-negativ), MCF-7 (Brustkrebszellinie, Östrogenrezeptor-positiv) und T-47-D (Brustkrebszellinie, Östrogenrezeptor-positiv) und humanen Fibroblasten (HGF) als Kontrollzellen wurde mRNA isoliert, durch Reverse Transkriptase die komplementäre cDNA hergestellt und die BSP-cDNA durch PCR mit BSP-spezifischen Primern amplifiziert. Die Expression von BSP-mRNA war in der Brustkrebszellinie MCF-7 besonders hoch, bei den MDA-MB-231 und T-47-D Zellen gering und bei der Kontrollzellinie nicht nachweisbar.

### Beispiel 10 - Herstellung von humanisierten monoklonalen Antikörpern

Der monoklonale Antikörper BSP 1.2 kann aufgrund seiner spezifischen Bindung an Tumor-BSP für die Therapie von Primärtumoren und Metastasen eingesetzt werden. Dabei bindet der Antikörper an BSP auf der Zelloberfläche bestimmter Tumorzellen und stimuliert das Immunsystem zur Zerstörung dieser Zellen z.B. über die Aktivierung der Komplementkaskade. Ähnlich lassen sich auch die polyklonalen oder monoklonale Anti-BSP IgY für die Therapie einsetzen. Beim Einsatz dieser Antikörper reagiert das menschliche Immunsystem mit der Bildung von eigenen Antikörper - humane Anti-Maus-IgG Antikörper (HAMAs) bzw. humane Anti-Huhn-IgY Antikörper (HACAs). HAMAs und HACAs können eine Immunantwort des Organismus auf das Tumorantigen induzieren und verstärken. Bei der Bestimmung von Tumormarkern entstehen jedoch Interferenzen mit den HAMAs und HACAs, die die in vitro Messmethode stören. Auf diese Weise kommt es zu falsch hohen Meßwerten für Tumormarker.

Deshalb eignen sich für die Therapie und Immunszintigraphie besonders humanisierte monoklonale Antikörper. Mehrere Verfahren sind beschrieben, wie man aus den Hybridomazelllinien, welche monoklonale Anti-BSP Antikörper produzieren, entsprechende humanisierte Antikörper ableitet.

### Beispiel 11 - Konjugate von Anti-BSP Antikörpern mit Zellgiften und Radioisotopen

In einer weiteren Anwendung der Erfindung können Zellgifte und Radioisotope chemisch kovalent mit den Anti-BSP Antikörper oder deren Fab-Fragmente verbunden werden. Mit Radioisotopen wie Jod 125 oder Jod 131 markierte Antikörper eignen sich bei Applikation geringer Mengen zur Tumorlokalisation über die Immunszintigraphie und bei Applikation großer Mengen zur direkten Zerstörung der Tumore. Solche chemischen Konjugate können zum Beispiel durch Jodierung der Antikörper mit Jod 125 oder 131 hergestellt werden (Garvey, J.S et al., Methods in Immunology. 3rd ed., W.A.Benjamin Publ., 1977, 171-182). Eine Übersicht über geeignete Verfahren zur Radioimmuntherapie und Immunszintigraphie findet sich bei Vuillez, Radioimmunotargeting: diagnosis and therapeutic use, in Bull Cancer. 2000, 87(11), 813-27.

### Beispiel 12 - Therapie von Tumoren mit Expression von BSP auf der Zelloberfläche

Es wurde an Biopsiematerial zunächst festgestellt, ob BSP auf der Oberfläche der Tumorzellen exprimiert wird. Patienten, bei denen BSP auf der Oberfläche der Tumorzellen nachweisbar ist, kommen für eine Therapie mit Anti-BSP Antikörpern des Huhns, der Maus, den entsprechenden humanisierten Antikörpern und mit Konjugaten dieser Antikörper mit Zellgiften oder Radioisotopen in Frage.

Die Behandlung von Tumoren mit therapeutischen Antikörpern, die gegen auf Zelloberflächen exprimierte Tumormarker gerichtet sind, ist Stand der Technik. So kann mit dem humanisierten Antikörper Herceptin gegen den Rezeptor für den humanen Epithelwachstumsfaktor Brustkrebs selbst in der metastasierten Form bei ca. 25 % der Betroffenen erfolgreich therapiert werden (Hotaling TE et al., The humanized anti-HER2 antibody rhuMAb HER2 mediates antibody dependent cell-mediated cytotoxicity via FcgR III [abstract]. Proc Annu Meet Am Assoc Cancer Res 1996; 37:47; Pegram MD et al., Antibody dependent cell-mediated cytotoxicity in breast cancer patients in Phase III clinical trials of a humanized anti-HER2 antibody [abstract]. Proc Am Assoc Cancer Res 1997; 38:602.

Ähnlich wie bei Herceptin kann der entsprechende Anti-BSP Antikörper als Infusion appliziert werden, z.B. als 90 Minuten Infusion bei der Erstapplikation und später als 30 Minuten Infusion. Die Häufigkeit der Infusionen und die Menge der Antikörper richten sich nach der Halbwertszeit der Antikörper im Blut (ca. 6 Tage bei einem humanisierten Antikörper und weniger als 24 Stunden bei einem Hühnerantikörper) und dem Körpergewicht.

### Beispiel 13 - Therapie von Tumoren durch Neutralisierung von freiem, nicht an Zellen gebundenen BSP und des BSP-Faktor-H Komplexes

Mit den oben beschriebenen Verfahren wurde festgestellt, dass die Tumorzellen des Patienten BSP exprimieren, das nicht auf der Zelloberfläche nachgewiesen werden kann. Bei diesen Tumoren kann davon ausgegangen werden, dass die Zellen BSP in das Blut oder die Gewebsflüssigkeit abgeben und z.B. zur Bindung von Faktor H zur Inaktivierung des alternativen Wegs der Komplementkaskade oder zur Einwanderung in Knochengewebe nutzen. Ein weiterer möglicher Indikator für diesen Tumortyp sind erhöhte Konzentrationen des BSP im Blutserum (> 20 ng / mL Serum). In diesen Fällen können Anti-BSP Antikörper zur Neutralisierung des freien bzw. mit Faktor H komplexierten Tumor-BSP eingesetzt werden. Die Dosis kann dabei auf die Menge des frei im Serum und in der Gewebsflüssigkeit vorhandenen BSP abgestimmt werden. Für die Therapie kommen Anti-BSP Antikörper aus dem Huhn, der Maus und humanisierte Anti-BSP Antikörper in Frage, die das freie BSP-Epitop im Komplex mit Faktor H erkennen können. Auch in Frage kommen Fab-Fragmente dieser Antikörper, die nach einem Standardverfahren durch proteolytischen Verdau präpariert werden können (Garvey, J.S et al., Methods in Immunology. 3rd ed., W.A.Benjamin Publ., 1977, 256 - 266). Auch gentechnisch hergestellte aus den obigen Anti-BSP Antikörpern abgeleitete Fab Fragmente kommen für eine solche Therapie in Frage.

Die Erfindung stellt somit Antikörper bereit gegen das humane Bone-Sialoprotein (hBSP), die spezifisch nur Epitope auf hBSP von Tumorzellen binden, denn Tumor-hBSP enthält keine posttranslationale O-Glykosylierung im Bereich der Aminosäuren 120 bis 135 (SWISSPROT: SIAL_HUMAN, Acc.No. P21815, ohne Signalsequenz), beinhaltend die Aminosäuren TGLAA. Anders das normale hBSP aus Knochen. Die Antikörper können tumorgenes Serum-hBSP im Komplex mit dem Komplementfaktor-H erkennen und bilden somit ein diagnostisch und therapeutisches wertvolles Instrument.

### Beispiel 14: Herstellung spezifischer Antikörper gegen BSP- oder andere Cluster des Tumorzelloberflächenproteoms.

Zur Herstellung spezifischer Antikörper gegen die genannten Proteine hat sich überraschenderweise gezeigt, dass neben der Benutzung von Gesamtmolekülen auch spezifische Aminosäuresequenzen von Epitopen besonders für die Immunisierung geeignet sind, wenn die synthetisierten Peptide nach den üblichen Verfahren an Trägermoleküle gekoppelt sind und bei Mäuse injiziert werden. Des weiteren eignen sich auch multiple antigene Peptide (Sequenzen siehe oben), die mittels Lysin zu größeren Molekülen verbunden sind, oder BSPtransfizierte Zelllinien, um diese Antikörper herzustellen. Als weitere Methode hat sich die Verwendung von Immunogenen aus stabil transfizierten, BSP-exprimierenden Zellen überraschend gut bewährt, wobei Membranisolate, Zellextrakte mit kompletten oder fragmentierten Rezeptoren oder auch lyophilisierte Gesamtpräparate benutzt wurden.

Die Mäuse (Typ NZW X NZB) wurden zur Herstellung monoklonaler Antikörper eingesetzt, was mit den Routinemethoden der Immundiagnostik AG und IPF PharmaCeuticals GmbH erfolgte. Die durch Western-Blot und ELISA überprüften Antikörper können nach Hochaufreiniguung für die genannten diagnostischen und therapeutischen Zwecke eingesetzt werden

### Beispiel 15: Einleitung der Apoptose durch BSP-spezifische Antikörper in BSP-exprimierenden Zelllinien.

Expressionsanalysen an verschiedene Zelllinien haben unter anderem gezeigt, dass Prostatatumor-Zelllinien, als auch Mammatumor-Zelllinien BSP exprimieren. Diese Expression wurde auf mRNA-Ebene mittels RT-PCR und auf Proteinebene mittels Western-Blot und FACS-Analyse durchgeführt. Eine Behandlung dieser BSP-exprimierenden Tumor-Zelllinien mit BSP-spezifischen Antikörpern hat in kultivierten Zellen zum programmierten Zelltod geführt, was unter anderem durchflusszytometrisch nachgewiesen werden konnte.

### Beispiel 16: Reduktion von Knochenmetastasen im Tiermodell.

Nach der Applikation von BSP-exprimierenden Tumor-Zelllinien in immundefizienten Nacktmäusen/-ratten kommt es regelmäßig zu einer Knochenmetastasierung. Bei gleichzeitiger Gabe des BSP-spezifischen Antikörpers kam es überraschenderweise zu einer deutlichen Reduktion der Ausbildung von manifesten Knochenmetastasen, was durch histologische Analyse der Gewebe belegt werden konnte.

### 1. Material und Methodik

Unser Tiermodell beinhaltete die Injektion von Brustkrebszellen und die anschließende Therapie der entstandenen lytischen Läsionen mit dem Anti-BSP-Antikörpern, welche ein Gemisch dargestellten von polyklonalen IgY-Antikörpern aus Huhn mit überwiegender Spezifität für humanes BSP aus Tumorzellen, die zudem hBSP in Humanserum in Gegenwart von Faktor-H quantitativ binden und die vorwiegend an ein Epitop im Bereich der Aminosäuren 120 bis 135 des hBSP binden, wobei die posttranslationale Glykosylierung in diesem Bereich bei hBSP aus Tumorzellen gegenüber natürlichem BSP aus Knochen verändert ist.

Injiziert wurden MDA-MB 231-Zellen (ATCC, HTB-26), die in einer vorausgegangenen Studie bei Nacktmäusen intracardial appliziert wurden (T.A.Guise, 1997; PTH-rP and Bone Metastases; American Cancer Society). Die Zelllinie wurde aus einem metastasiertem humanen Adenocarcinom gewonnen, sie besitzen zudem keine Östrogenrezeptoren. In unserem Falle war sie mit Green-Fluorescent Protein (GFP) markiert, was die Auffindung der Zellen im histologischen Präparat erleichtert. Als Versuchstiere wurden Nacktratten im Alter von 6 bis 8 Wochen (RNU, Charles River Breeding, Sulzfeld, Deutschland) verwendet, die in ihrer Immunkompetenz gemindert sind, so dass die injizierten humanen Zellen nicht als solche erkannt und bekämpft wurden. Unsere Voruntersuchungen mit verschiedenen Zellzahlen bei Männchen und Weibchen ergaben, dass die Metastasen bei männlichen Ratten nach Injektion von 10⁵ MDA-MB 231-Zellen nach ungefähr einem Monat in Form von lytischen Läsionen sichtbar werden.

Die Zellen (1 x 10⁵ in PBS-Puffer) wurden intraarteriell in die A. femoralis gespritzt (o,2 ml; n=8). Hierzu wurde ein Seitenast dieses Gefäßes kanüliert und nach der Injektion abgebunden, um ein Austreten der eingebrachten Zellen zu verhindern. Das Tier kompensiert den Verlust des Gefäßes durch Kollateralenbildung problemlos. Die Krebszellen gelangen dann mit dem Blut zu den feinen Aufzweigungen der versorgenden Äste von Femur, Tibia und Fibula. Es findet hier in der terminalen Strombahn Extravasation und drauffolgende Adhäsion der Zellen an die Knochenmatrix statt. Hier erfolgt wahrscheinlich auch die Interaktion mit BSP.

Die anschließende Überwachung des Metastasenwachstums erfolgte 10-tägig mit konventionellen Röntgenaufnahmen anterior-posterior und posterior-anterior unter Ethernarkose des Tiers. Die ungefähre Quantifizierung wurde durch Vermessung der Läsion in Länge und Breite ihrer Ausdehnung vorgenommen.

Nach zweimalig positiver Röntgenkontrolle wurden die Tiere mit Anti-BSP-Ak oder Standard therapiert. Die Therapie erfolgte bei den beschriebenen Tieren einmalig pro Woche subcutan in einer Konzentration von 10 mg/kg Körpergewicht.

Die Nachbeobachtung der Tiere erfolgte ferner durch Computertomographie und Histologie (noch nicht abgeschlossen). Die CT erlaubte eine dreidimensionele Rekonstruktion des Knochens und des Defekts sowie eine exakte Vermessung der Läsionsgröße. Nach der Beobachtungszeit wurden die Tiere getötet und histologisch untersucht. Durch die Markierung mit GFP sind die Zellen unter UV-Licht im Knochenschnittpräparat sichtbar. Außerdem können histologisch genauere Aussagen über den vorausgegangenen Umbau des Knochengewebes getroffen werden.

### 2. Ergebnisse

Die Ergebnisse sind in den nachstehenden Abbildungsreihen 4 und 5 beispielhaft dokumentiert. Die Tiere wurden nach der Operation mehr als 3 Monate röntgendiagnostisch (Siemens Opti 150/30/50 HC-100) beobachtet. Bei den Kurvenverläufen ist darauf zu achten, dass die auf der Ordinate aufgetragenen Flächenangaben der Läsionsgrößen in mm² bei den beiden gezeigten Tieren unterschiedlich sind. Insgesamt wurden 8 Tiere therapiert.

Die Tiere 987 und 988 (siehe Abbildungen) wurden subcutan mit dem Anti-BSP-Ak (10 mg/kg) einmal wöchentlich behandelt. Die Gesamtdauer der Behandlung betrug ca. 50 Tage. Die Therapie wurde nach 2 bzw. 3 positiven Röntgenkontrollen begonnen. Dies entspricht bei Tier 987 Tag 46 nach der Operation und bei Tier 988 dem 32. postoperativen Tag.

Einer raschen Läsionsgrößenzunahme ab dem 24. postoperativen Tag bei Tier 988 folgte eine fortschreitende Lyse des Knochens, die ab Tag 38 zu einer Fraktur im mittleren Drittel der Tibia führte. Diese fand unter begonnener Therapie (ab Tag 32) statt. Erste Heilungstendenzen zeigten sich ab dem 52. Tag in Form von Kallusbildung an der Frakturstelle und auch die Läsionen an der proximalen Tibia und dem distalen Femur wurden kleiner. Die äußeren Begrenzungen der Läsionen waren anfangs scharf abgegrenzt, später während der Heilung zunehmend unschärfer begrenzt. Die Neubildung des Knochens erfolgte vom äußeren Rand her ausgehend hin zum Zentrum der Läsion. Ab Tag 89 war die Läsionsgröße nur schwer quantifizierbar, da durch die zunehmende Kallusbildung die genauen Ränder nicht mehr erkennbar waren. Nach dem 126. Tag kann man von einer Vollremission sprechen, wie das beigelegte Bild zeigt (komplettes Verschwinden der röntgenologisch erkennbaren lytischen Läsion). Die dreidimensionalen CT-Rekonstruktionen (Siemens Somato Plus 4, Volume Zoom) zeigen die Veränderungen der Tibia-Läsion nach 31 und 80 Tagen post OP. Es ist eine deutliche Zunahme an Knochengewebe sichtbar.

Das Tier 987 wurde nach 3 positiven Röntgenbefunden auch mit 10 mg/kg s.c. einmal wöchentlich ab Tag 46 therapiert. Hier zeigte sich nur eine Metastase am distalen Femur, die schon ab dem 89. postoperativen Tag (also nach 42 Tagen unter Therapie) knöchern verheilt war.

Andere Tiere wurden unter gleichen Bedingungen bis zu fünfmal pro Woche mit 10 mg/kg s.c. und bis zu zweimal pro Woche 10 mg/kg i.v. therapiert. Es war aber eine Größenzunahme der Läsion während und nach Beendigung der Therapie zu beobachten, wenn durch die Behandlung eine Immunreaktion gegen die injizierten BSP-Antikörper induziert wird.

Vergleichend wurden Tiere mit dem Alkylphosphocholin Er-PC₃ mit 40 ìMol/kg i.v. zweimal wöchentlich therapiert, das mit dieser Konzentration primäre Mamakarzinome regredieren lässt (Positivkontrolle), aber bei Knochenmetastasen keine Wirkung zeigt [Berger, M.R. et al., (1998) Erucylphosphocholine is the prototype of i.V. injectable alkylphosphocholines. Drugs of Today, 34 (Sppl. F), 73-81] . Hier zeigte sich nach Beendigung der Therapie bei einem Tier keine Veränderung, bei einem anderen sogar eine Verschlechterung der Situation (Progression) im Vergleich zum Therapiebeginn.

Gegenstand der vorliegenden Erfindung ist somit ein Arzneimittel, enthaltend Antikörper oder Bindemoleküle wie Aptamere gegen tumorspezifisches BSP oder andere Liganden für das gleiche Protein. Weiter kann die Verwendung der vorgeschlagenen Arzneianwendung durch Einsatz folgender Substanzen verstärkt werden: Antikörper, Liganden oder Inhibitoren die mit Adhäsionsmolekülen, membran-assoziierten Proteasen, oder Rezeptoren, die Chemotaxis vermitteln, wie beispielsweise Chemokinrezeptoren, interagieren, sowie Apoptose-induzierende Substanzen wie vorzugsweise Antikörper oder Proteine/Peptide, die aus natürlichen oder künstlichen Peptidbanken gewonnen werden können. Das Arzneimittel kann einzeln oder in Kombination mit oben genannten Substanzen insbesondere zur Therapie von Tumorerkrankungen, vorzugsweise deren Knochenmetastasen, angewendet werden.

Die Erfindung betrifft weiterhin ein Verfahren zur Therapie und der medizinischen und gewerblichen Verwendung der genannten Antikörpern gegen BSP oder andere Liganden für das gleiche Protein bzw. deren Kombination mit verstärkenden Antikörpern, Liganden oder Inhibitoren die mit Adhäsionsmolekülen, membran-assoziierten Proteasen, oder Rezeptoren die Chemotaxis vermitteln, wie beispielsweise Chemokinrezeptoren, interagieren, sowie Apoptose-induzierende Substanzen wie vorzugsweise Antikörper oder Proteine/Peptide, die aus natürlichen oder künstlichen Peptidbanken gewonnen werden können, um das Krebswachstum inklusive Metastasierung zu hemmen. Das Verfahren beruht auf der Feststellung, dass BSP über die krankheitsspezifische Konstellation der Expression auf spezifische Tumorzellen wirken kann. Primär- und Sekundärtumore werden unter anderem durch BSP in ihrem Migrations- und Proliferationsverhalten gesteuert. Daraus ergibt sich die Möglichkeit, das Krebswachstum sowie die Tumormetastasierung mittels genanntem Verfahren/Therapie entscheidend zu verhindern oder vollständig zu unterdrücken.

## Patentansprüche

1. Antikörper, Immuntoxine, Moleküle mit BSP-Bindungsstruktur von natürlichen BSP-Rezeptoren oder Faktor-H-Molekülen, und Aptamere oder Spiegelmere auf DNA- oder RNA-Basis, welche Bone-Sialoprotein oder Fragmente hiervon in Serum oder Plasma binden, zur Verwendung als Wirkstoff zur Behandlung von Knochenmetastasen und BSP-exprimierenden Tumoren, die bevorzugt in Knochengeweben ansiedeln.

2. Wirkstoffe gemäß Anspruch 1, die spezifisch humanes Bone-Sialoprotein (hBSP) oder Fragmente hiervon in Serum oder Plasma binden.

3. Wirkstoffe gemäß Anspruch 1 oder 2, die spezifisch Bone-Sialoprotein aus Tumorzellen binden.

4. Wirkstoffe gemäß einem der vorstehenden Ansprüche, hergestellt gegen chemisch oder natürlich in der Glykosylierung verändertes Bone-Sialoprotein oder einem Fragment hiervon.

5. Wirkstoffe nach Anspruch 4, hergestellt gegen Bone-Sialoprotein aus Knochenmaterial, dessen Spender nicht zur normalen Glykosylierung von Knochenproteinen fähig ist.

6. Wirkstoffe nach einem der vorstehenden Ansprüche, die spezifisch humanes Bone-Sialoprotein (hBSP) binden, dessen posttranslationale Glykosylierung im Bereich der Aminosäuren 120 bis 135 (SWISSPROT: SIALJHUMAN, Acc.No. P21815), beinhaltend die Aminosäuren TGLAA, gegenüber normalem Bone-Sialoprotein aus Knochen verändert oder unvollständig ist.

7. Wirkstoffe nach einem der vorstehenden Ansprüche, hergestellt gegen ein hBSP-Epitop, umfassend die Aminosäuresequenz TGLAA oder YTGLAA und optional Zuckergruppen.

8. Wirkstoffe nach einem der vorstehenden Ansprüche, hergestellt gegen ein hBSP-Antigen mit der Aminosäuresequenz YTGLAAIQLPKKAGD (SEQ ID NR: 1).

9. Wirkstoff nach einem der vorstehenden Ansprüche, wobei der Wirkstoff ein IgY-Antikörper aus Huhn ist.

10. Wirkstoff nach Anspruch 9, wobei der Antikörper human oder humanisiert ist.

11. Wirkstoff nach einem der vorstehenden Ansprüche, wobei der Wirkstoff ein bispezifischer Antikörper ist, der ein zusätzliches Paratop beinhaltet, das vorzugsweise spezifisch für Epitope von CD3 ist.

12. Wirkstoff nach einem der vorstehenden Ansprüche, wobei der Wirkstoff ein Immuntoxin ist, welches ein Konjugat aus Bindemolekül und einem Rest mit zytotoxischer Aktivität ist.

13. Wirkstoff nach Anspruch 11, wobei das Konjugat die Ricin-A-Kette oder ein nicht bindendes Fragment des Diphtherietoxins beinhaltet.

14. Wirkstoff nach einem der vorstehenden Ansprüche, wobei der Wirkstoff mit einem Radionuklid gekoppelt ist.

15. Pharmazeutische Zusammensetzung zur Behandlung von Erkrankungen durch Tumore und deren Metastasen, die bevorzugt in Knochengeweben ansiedeln, enthaltend einen Wirkstoff nach einem der Ansprüche 1 bis 14.

16. Pharmazeutische Zusammensetzung nach Anspruch 15, enthaltend mindestens einen weiteren Antikörper, Liganden oder Inhibitor, ausgewählt aus Adhäsionsmoleküle, membranassoziierte Proteasen, Chemotaxis vermittelnden Rezeptoren, Chemokinrezeptoren, Apoptose-induzierenden Substanzen.

17. Pharmazeutische Zusammensetzung nach Anspruch 16, wobei die Inhibitoren zumindest teilweise BSP blockieren und somit dessen Funktion modulieren.

18. Wirkstoff oder pharmazeutische Zusammensetzung nach einem der vorhergehenden Ansprüche zur Behandlung von BSP-exprimierenden Tumoren aus der Gruppe Prostata-, Mamma-, Lungen-, Nieren- und Schilddrüsentumoren, Tumorerkrankungen des Blutsystems, des Lymphsystems, des Herz-Kreislauf-Systems, des Nervensystems, des Respirationstraktes, des Verdauungstraktes, des endokrinen Systems, der Haut einschließlich Anhangsgebilde, des Bewegungsapparates und des Urogenitaltraktes, einschließlich der Niere.

## Claims

1. Antibodies, immunotoxins, molecules with a BSP-binding structure of natural BSP-receptors or of factor H molecules, and aptamers or spiegelmers on the basis of DNA or RNA which bind bone sialoprotein or fragments thereof in serum or plasma for use as an active ingredient in the treatment of bone metastases and BSP-expressing tumors that preferentially settle in bone tissues.

2. Active ingredients according to claim 1 which specifically bind human bone sialoprotein (hBSP) or fragments thereof in serum or plasma.

3. Active ingredients according to claim 1 or 2 which specifically bind bone sialoprotein from tumor cells.

4. Active ingredients according to any previous claim, produced against bone sialoprotein which has been modified chemically or naturally in the glycosylation, or a fragment thereof.

5. Active ingredients according to claim 4, produced against bone sialoprotein isolated from bone material, the donor of which is incapable of normal glycosylation of bone proteins.

6. Active ingredients according to any of the previous claims which specifically bind human bone sialoprotein (hBSP) which posttranslational glycosylation in the region of amino acids 120 to 135 (SWISSPROT: SIALJHUMAN, Acc. No. P21815), comprising amino acids TGLAA, is modified or incomplete compared to normal bone sialoprotein from bones.

7. Active ingredients according to any previous claim produced against a hBSP epitope comprising the amino acid sequence TGLAA or YTGLAA and optionally sugar groups.

8. Active ingredients according to any previous claim produced against a hBSP antigen comprising the amino acid sequence YTGLAAIQLPKKAGD (SEQ. ID NR: 1).

9. Active ingredient according to any previous claim wherein the active ingredient is an IgY antibody from chicken.

10. Active ingredient according to claim 9 wherein the antibody is human or humanised.

11. Active ingredient according to any previous claim wherein the active ingredient is a bispecific antibody comprising an additional paratope which is preferably specific for epitopes of CD3.

12. Active ingredient according to any previous claim wherein the active ingredient is an immunotoxin which is a conjugate of a binding molecule and a moiety with cytotoxic activity.

13. Active ingredient according to claim 11 wherein the conjugate comprises the ricin-A-chain or a non-binding fragment of the diphtheria toxin.

14. Active ingredient according to any previous claim wherein the active ingredient is coupled to a radionuclide.

15. Pharmaceutical composition for treatment of conditions caused by tumors and their metastases which preferentially settle in bone tissues, comprising an active ingredient according to any of claims 1 to 14.

16. Pharmaceutical composition according to claim 15 comprising at least one further antibody, ligand or inhibitor, selected from adhesion molecules, membrane associated proteases, chemotaxis mediating receptors, chemokine receptors, apoptosis inducing substances.

17. Pharmaceutical composition according to claim 16 wherein the inhibitors at least partially block BSP and hence modulate its function.

18. Active ingredient or pharmaceutical composition according to any previous claim for a treatment of BSP-expressing tumors selected from tumors of the prostate, breast, lung, kidney and thyroid, the circulatory system, lymphoid system, cardiovascular system, nervous system, respiratory tract, digestive tract, endocrine system, skin including adnexa, muscoskeletal system and urogenital system, including kidneys.

## Revendications

1. Anticorps, immunotoxines, molécules contenant une structure de liaison de récepteurs naturels de sialoprotéine osseuse ou de molécules de facteur H, et aptamères ou spiegelmères sur la base d'ADN ou d'ARN, qui lient de la sialoprotéine osseuse ou ses fragments dans du sérum ou du plasma, pour utilisation comme agent actif pour le traitement de métastases osseuses et de tumeurs exprimant de la sioaloprotéine osseuse qui s'implantent préférentiellement dans des tissus osseux.

2. Agents actifs selon la revendication 1 qui lient spécifiquement de la sialoprotéine osseuse humaine (hBSP) ou ses fragments dans du sérum ou du plasma.

3. Agents actifs selon la revendication 1 ou 2 qui lient spécifiquement de la sialoprotéine osseuse de cellules tumorales.

4. Agents actifs selon l'une des revendications précédentes produits contre de la sialoprotéine osseuse modifiée chimiquement ou naturellement dans la glycosylation ou un de ses fragments.

5. Agents actifs selon la revendication 4 produits contre de la sialoprotéine osseuse issue de matériel osseux dont le donneur est incapable de la glycosylation normale de protéines osseuses.

6. Agents actifs selon l'une des revendications précédentes qui lient spécifiquement de la sialoprotéine osseuse humaine (hBSP) dont la glycosylation posttranslationale dans la région des acides aminés 120 à 135 (SWISSPROT : SIALJHUMAN, Acc.No. P21815), qui comprennent des acides aminés TGLAA, est variée ou incomplète par rapport à de la sialoprotéine osseuse normale issue des os.

7. Agents actifs selon l'une des revendications précédentes produits contre un épitope de hBSP comprenant la séquence d'acides aminés TGLAA ou YTGLAA et optionnellement des groupes de sucre.

8. Agents actifs selon l'une des revendications précédentes produits contre un antigène de hBSP avec la séquence d'acides aminés YTGLAAIQLPKKAGD (SEQ ID NR : 1)

9. Agent actif selon l'une des revendications précédentes dans lequel l'agent actif est un anticorps IgY de poule.

10. Agent actif selon la revendication 9 dans lequel l'anticorps est humain ou humanisé.

11. Agent actif selon l'une des revendications précédentes dans lequel l'agent actif est un anticorps bispécifique comprenant un paratope additionnel qui est préférentiellement spécifique pour des épitopes de CD3.

12. Agent actif selon l'une des revendications précédentes dans lequel l'agent actif est une immonotoxine qui est un conjugué d'une molécule de liage et d'un reste ayant une activité cytotoxique.

13. Agent actif selon la revendication 11 dans lequel le conjugué comprend la chaîne ricine-A ou un fragment non-liant de la toxine de diphtérie.

14. Agent actif selon l'une des revendications précédentes dans lequel l'agent actif est couplé à un radionucléide.

15. Composition pharmaceutique pour le traitement de maladies de tumeurs et de leurs métastases qui s'implantent préférentiellement dans des tissus osseux, comprenant un agent actif selon l'une des revendications 1 à 14.

16. Composition pharmaceutique selon la revendication 15 comprenant au moins un anticorps, un ligand ou un inhibiteur additionnel, choisi parmi des molécules d'adhésion, des protéases associées à des membranes, des récepteurs causant la chimiotaxie, des récepteurs de chimiokines, des substances induisant l'apoptose.

17. Composition pharmaceutique selon la revendication 16 dans laquelle les inhibiteurs bloquent au moins partiellement la sialoprotéine osseuse et modifient ainsi sa fonction.

18. Agent actif ou composition pharmaceutique selon l'une des revendications précédentes pour le traitement de tumeurs exprimant la sialoproténe osseuse du groupe avec des tumeurs de la prostate, du sein, du poumon, du rein et de la thyroïde, du système circulaire, du système lymphoïde, du système cardiovasculaire, du système nerveux, des voies respiratoires, de l'appareil digestif, du système endocrinien, de la peau y compris ses annexes, de l'appareil locomoteur et du système urogénital, y compris le rein.
